# EUROPEAN PATENT APPLICATION

(11) **EP 0 999 284 A1**
(43) Date of publication of application: **10.05.2000**
(21) Application number: 98402255.8
(22) Date of filing: 11.09.1998
(51) Int. Cl.: C12Q 1/68, C12Q 1/18

(54) **Method for screening antimycotic substances using essential genes from c.albicans, and said genes**

(71) Applicant: HOECHST MARION ROUSSEL, 92800 Puteaux (FR)
(72) Inventor: The designation of the inventor has not yet been filed
(74) Representative: Pochart, François

(57) **Abstract**

The present invention concerns a method for the screening of antimycotic substances wherein an essential gene from mycetes or a functionally similar mycete gene, or the corresponding encoded protein, is used as target and wherein the essential gene is selected from the group consisting of CaNL256, CaBR102 and CaIR012.

The invention also concerns these genes and plasmids containing same.

## Description

The present invention relates to a method for screening for antimycotic substances in which essential genes from mycetes, particularly from Candida albicans (C.albicans) as well as functionally similar genes from other pathogenic mycetes, or the corresponding encoded proteins, are used as targets. The invention also relates to specific C. albicans genes.

The spectrum of known fungal infections stretches from fungal attack of skin or nails to potentially hazardous mycotic infections of the inner organs; Such infections and resulting diseases are known as mycosis.

Antimycotic substances (fungistatic or fungicidal) are used for treatment of mycosis. However, up to now, relatively few substances with pharmacological effects are known, such as Amphotericin B, Nystatin, Pimaricin, Griseofulvin, Clotrimazole, 5-fluoro-cytosine and Batraphene. The drug treatment of fungal infections is extremely difficult, in particular because both the host cells and the mycetes, are eucaryotic cells. Administration of drugs based on known antimycotic substances results therefore often in undesired side-effects, for example Amphotericin B has a nephrotoxic effect. Therefore, there is a strong need for pharmacologically efficient substances usable for the preparation of drugs, which are suitable for prophylactic treatments of immunodepressive states or for the treatment of an existing fungal infection. Furthermore, the substances should exhibit a specific spectrum of action in order to selectively inhibit the growth and proliferation of mycetes without affecting the treated host organism.

The aim of the present invention is to provide a method for the identification of antimycotic substances and especially for the identification of anti-Candida substances. An essential feature of this method is that essential genes from mycetes are used as targets for the screening.

The present invention thus concerns a method for screening antimycotic substances wherein an essential gene from mycetes or a functionally similar gene in another pathogenic mycete, or the corresponding encoded protein, is used as target and wherein the essential gene is selected from the group consisting of CaNL256, CaBR102 and CaIR012.

According to one embodiment of the method of the invention mycete cells which express the essential gene, or a functionally similar mycete gene, to a different level are incubated with the substance to be tested and the growth inhibiting effect of the substance is determined.

According to another embodiment, said target gene or the corresponding target gene encoded protein is contacted in vitro with the substance to be tested and the effect of the substance on the target is determined.

According to another embodiment, the screened substances inhibit partially or totally the functional expression of the essential genes or the functional activity of the encoded proteins.

According to one embodiment the screened substances partially or totally inhibit the activity of dihydropteroate synthase (DHPS) and/or 7,8-dihydro-6-hydroxymethylpterin-pyrophosphokinase (HPPK).

According to another embodiment, the mycete species are selected from the group comprising Basidiomycetes, Ascomycetes and Hyphomycetes.

According to another embodiment of the method of the invention said functional similar genes are essential genes from Candida Spp., preferably Candida albicans, or from Aspergillus Spp., preferably from Aspergillus fumigatus.

According to another aspect, the present invention concerns a gene having the sequence of CaNL256, CaBR102 or CaIR012, identified as SEQ ID NO:2, SEQ ID NO:4, SEQ ID NO:6, respectively, or a functionally similar gene or a functional fragment thereof.

According to this embodiment, the functionally similar gene has a sequence identity, at the nucleotide level, with CaNL256, CaBR102 or CaIR012, respectively, of at least 50%, preferably of at least 60%, and most preferably of at least 70%.

According to another embodiment, the functionally similar gene has a sequence identity, at the amino-acid level, with CaNL256, CaBR102 or CaIR012, respectively, encoded protein(s) of at least 40%, preferably of at least 50%, more preferably of at least 60% and most preferably of at least 70%.

According to another aspect, the present invention covers the protein(s) encoded by the CaNL256, CaBR102 or CaIR012, respectively, gene(s) or a functional polypeptidic fragment thereof.

According to another aspect, the present invention provides a plasmid containing the CaNL256, CaBR102 or CaIR012, respectively, gene(s), a functionally similar gene or a functional fragment thereof

According to another aspect, the present invention provides a plasmid (bacteria containing same) deposited at the CNCM (Institut Pasteur, Paris) with the accession numbers I-2065, I-2063 and I-2064, corresponding to the CaNL256, CaBR102 and CaIR012 genes, respectively.

According to another aspect, the present invention provides a kit for diagnosis of fungal infections comprising a gene selected from the group consisting of CaNL256, CaBR102 and CaIR012, a functionally similar gene thereof, a functional fragment thereof, the corresponding encoded protein or a functional polypeptide fragment thereof.

According to another aspect, the present invention provides an antibody directed against the protein encoded by the CaNL256, CaBR102 or CaIR012, respectively, gene(s) or a polypeptide fragment thereof.

According to the invention, it is thus necessary first of all to identify said essential genes and starting from these thus identified genes, essential genes from other pathogenic mycetes can be identified. For practical purposes, essential genes from S. cerevisiae are first identified and starting from them, essential genes from other pathogenic fungus, especially from Candida, are obtained.

The present invention thus discloses the identification of three essential genes from C.albicans and their use in a method for the screening of antimycotic substances, especially anti-Candida substances.

In order to identify essential genes of S.cerevisiae, individual genomic genes are eliminated through homologous recombination. If the DNA segment thus eliminated concerns an essential gene, then the deletion is lethal for the S.cerevisiae cells in haploid form.

A method, wherein the studied S.cerevisiae gene is replaced by a marker gene can be used to generate the corresponding genomic deletion of S.cerevisiae and to determine the S.cerevisiae cells containing the deletion.

As a selection marker a dominant selection marker (e.g. kanamycin resistance gene) or an auxotrophic marker can for example be used. As an auxotrophic marker, it is possible to use genes coding for key enzymes of amino acid or nucleic base synthesis. For example, one can use as a selection marker the following genes from S.cerevisiae : gene encoding for the metabolic pathway of leucine (e.g.LEU2-gene), histidine (e.g. HIS3-gene) or tryptophan (e.g. TRP-1 gene) or for the nucleic base metabolism of uracil (e.g. URA3-gene).

Auxotrophic S.cerevisiae strains can be used. These auxotrophic strains can only grow on nutritive media containing the corresponding amino acids or nucleotide bases. All laboratory S.cerevisiae strains, containing auxotrophic markers can for instance be used. When diploid S.cerevisiae strains are used, then the corresponding marker gene must be homozygously mutated. Strain CEN.PK2 or isogenic derivates thereof can be used.

Strains containing no suitable auxotrophic marker can also be used such as prototrophic S.cerevisiae strains. Then a dominant selection marker e.g. resistance gene, such as kanamycin resistance gene can be used. A loxP-KanMX-loxP cassette can advantageously be used for this purpose.

For the homologous recombination replacing the whole DNA sequence or part thereof of a S.cerevisiae gene, DNA fragments are used wherein the marker gene is flanked at the 5'- and 3'-ends by sequences which are homologous to the 5'- and 3'-ends of the studied S.cerevisiae gene.

Different processes can be used for the preparation of the corresponding DNA fragments which are also appropriate for the deletion of any specific S.cerevisiae gene. A linear DNA-fragment is used for the transformation of the suitable S.cerevisiae strain. This fragment is integrated into the S.cerevisiae genome by homologous recombination. These processes include:
1. "Conventional method" for the preparation of deletion cassettes (Rothstein, R.J. (1983) Methods in Enzymology, Vol. 101, 202-211).
2. "Conventional Method" using the PCR technique ("modified conventional method").
3. SFH (short flanking homology)- PCR method (Wach, A. et al. (1994) Yeast 10: 1793-1808; Gültner, U. et al. (1996) Nucleic Acids Research 24:2519-2524).

1. In the "conventional method" for the preparation of deletion cassettes in the S.cerevisiae genome, the gene to be studied is either already present in an appropriate vector or is integrated in such a vector. With this method, any pBR- pUC- and pBluescript®-derivates can be used for example. A major part of the target gene sequence is eliminated from the vector, for instance using appropiate restriction sites, conserving however the 3'- and 5'-regions of the studied gene inside the vector. The selected marker gene is integrated between the remaining regions.
2. In the modified form of this "conventional method", PCR is used. This method allows amplification of the 3'- and 5'-terminal regions of the coding sequence of the studied S.cerevisiae gene. This method amplifies selectively both terminal regions of the studied gene, therefore, two PCR-reactions must be carried out for each studied gene, amplifying once the 5'-end, and once the 3'-end of the gene. The length of the amplified terminal DNA-fragment depends on the existing restriction sites. The amplified terminal ends of the studied gene have generally a length of 50 to 5000 base pairs (bp), preferably a length between 500 and 1000 bp.
   As template for the PCR-reactions, genomic DNA of S.cerevisiae or wild-type genes can be used. The primer-pairs (a sense and an antisense primer, respectively) are constructed so that they correspond to the 3'-end and the 5'-end sequence of the studied S.cerevisiae gene. Especially, the primer is selected such as to allow its integration by way of appropriate restriction sites.
   As vectors, pBR- pUC- and pBluescript®-derivates can be used. In particular vectors already containing a gene encoding the selection marker, are appropriate. In particular, vectors can be used, which contain genes of the selection marker HIS3, LEU2, TRP1 or URA3.
   The DNA segments of the studied S.cerevisiae gene, obtained by PCR, are integrated in the vector at both sides of the selection marker, so that subsequently, as in the "conventional method", the selection marker is flanked on both ends by DNA sequences which are homologous to the studied gene.
3. Homologous recombination in S.cerevisiae takes place in a very efficient and precise manner and the length of the DNA sequence homologous to the studied S.cerevisiae gene flanking the selection marker gene can in fact be considerably shorter than with the "modified conventional method". The flanking ends homologous to the studied S.cerevisiae gene need to present a length of only about 20-60 bp, preferably 30-45 bp. The SFH-PCR method is particularly advantageous as the laborious cloning step can be obviated.

A PCR reaction is carried out on a DNA-template containing the gene for the selection marker to be used, wherein the primers are constructed so that the DNA sequence of the sense primer is homologous to the 5'-end of the selection marker sequence and so that the primer presents in addition at its 5'-end a region of preferably 40 nucleotides, which corresponds to the 5'-terminal sequence of the studied S.cerevisiae gene. The antisense primer is constructed in an analogous manner, i.e. it is complementary to the 3'-end of the gene sequence of the selection marker, wherein this primer contains at its 5'-end a region of also preferably 40 nucleotides, which corresponds to the complementary strand of the 3'-terminal coding sequence of the studied gene.

For the amplification of S.cerevisiae genes to be studied by the SFH-PCR method, vectors containing the gene for the auxotrophic marker or selection marker can be used. Especially, plasmid pUG6 is used as the template. This plasmid contains a loxP-KanMX-loxP cassette (Gültner, U. et al. (1996) Nucleic Acids Research 24: 2519-2524). In other terms, the Kanamycin resistance gene is flanked at both ends by a loxP sequence (loxP-KanMX-loxP cassette). This cassette is advantageous in that the Kanamycin resistance gene can be eventually eliminated from the S.cerevisiae genome after integration of the loxP-KanMX-loxP cassette into the S.cerevisiae gene to be studied. Cre-recombinase of bacteriophage P1 can be used for this purpose. Crerecombinase recognizes the loxP sequences and induces elimination of the DNA located between the two loxP sequences by a homologous recombination process. As a result only one loxP sequence remains and the so-called marker regeneration occurs, i.e. the S.cerevisiae strain may be transformed again using the loxP-KanMX-loxP cassette. This is particularly advantageous, when at least two functionally similar genes are to be deleted in order to obtain a lethal phenotype.

With the PCR-method, the PCR reaction primers are at the 3'-end a preferably 20 nucleotide long sequence, which is homologous to the sequence situated left and/or right of the loxP-KanMX-loxP cassette, and at the 5'-end a preferably 40 nucleotide long sequence, which is homologous to the terminal ends of the gene to be studied.

Using the three methods, one obtains linear deletion cassettes containing the gene encoding the selection marker, which is flanked on both sides by homologous sequences of the gene to be studied. The deletion cassettes are used for the transformation of diploid S.cerevisiae strains. The diploid strain S.cerevisiae CEN.PK2 (Scientific Research & Development GmbH, Oberursel) can be used for example for this purpose. [CEN.PK2 Mata/MAT α ura3-52/ura3-52 leu2-3, 112/leu2-3, 112his3Δ1/hisBΔ1 trp1-289/trp1-289 MAL2-8^{c}/MAL2-8^{c} SUC2/SUC2]

The strain CEN.PK2 is prepared and cultivated using known methods (Gietz, R.D. et al. (1992) Nucleic Acids Research 8: 1425; Güldener, U. et al. (1996) Nucleic Acids Research 24:2519-2524).

The cells of the S.cerevisiae strain used are transformed according to known processes with an appropriate DNA quantity of the linear deletion cassette (e.g. Sambrook et al. 1989). Thereafter, the medium in which the cells are cultivated is replaced by a new medium, a so-called selective medium, which does not contain the corresponding amino acid (e. g. histidine, leucine or tryptophan) or nucleic base (e. g. uracil) or, when using a deletion cassette containing the kanamycin resistance gene, by a medium containing geneticin (G418®) (e.g. a complete medium (YEPD) containing geneticin). Alternatively, the transformed cells may be plated on agar plates prepared using the corresponding media. Thereby, one selects the transformed cells, in which a homologous recombination occured, since only those cells can grow under these modified conditions.

However, in most cases, only one of the two copies of the gene in the double chromosome set of a diploid S.cerevisiae strain is replaced by the DNA of the deletion cassette during the transformation, resulting in a heterozygote-diploid S.cerevisiae mutant strain, wherein one copy of the gene studied is replaced by a selection marker, while the other copy of the gene is maintained in the genome. This presents the advantage that in case of a deletion of an essential gene, due to the existence of the second copy of the essential gene, the mutant S.cerevisiae strain is still viable.

The proper integration of the deletion cassette DNA at the predetermined chromosomal gene locus (gene locus of the gene to be studied) may be checked by Southern-Blot Analysis (Southern, E.M. (1975) J. Mol. Biol. 98:503-517) or by diagnostic PCR analysis using specific primers (Güldener, U. et al. (1996) Nucleic Acids Research 24:2519-2524)

The genetic separation of individual diploid cells may be monitored by tetrad analysis. To this end, reduction division (meiosis) is induced in the diploid cells, especially heterozygote mutant strains, using known methods such as nitrogen impoverishment on potassium acetate plates (Sherman, F. et al. (1986) Cold Spring Harbor Laboratory Press, Cold Spring Harbor, N. Y.; Guthrie, C. and Fink, G.R. (1991) Methods in Enzymology, Vol 194. Academic Press, San Diego, 3-21; Ausubel, F. M. et al. (1987) Current Protocol in Molecular Biology John Wiley and Sons, Inc., Chapter 13). Meiosis results only in asci with four ascospores (segregated), which can be indivualized after partial enzymatic digestion of the ascospore wall with zymolyase (Ausubel et al. (1987)) by way of micromanipulators (e.g. SINGER). For example when a tetrad analysis is carried out on a heterozygote-diploid mutant strain in which an essential gene present in the double chromosome set is replaced on one chromosome by homologous recombination, then only two segregated ascospores are viable, namely those which carry the essential gene. The two remaining segregated ascospores are not viable because they lack the essential gene.

In order to check if the genes studied by this method are really essential or if the homologous recombination leads to an alteration of an essential gene adjacent to the gene locus of the gene studied, the heterozygote diploid S.cerevisiae mutant strain is transformed with a centromere plasmid containing said studied gene.

A tetrad analysis is carried out on the transformants. When four instead of two viable segregates are obtained, then the studied gene contained in the centromere plasmid can complement the defect of the two non-viable haploid S.cerevisiae cells/mutant strains, which demonstrates that the studied S.cerevisiae gene is essential.

Preferably, plasmids present in low copy number, e.g. one or two copies per cell are used as centromere plasmids. For example plasmids pRS313, pRS314, pRS315 and pRS316 (Sijkorski, R. S. and Hieter, P. (1989) Genetics 122: 19-27) or similar plasmids can be used for this purpose. Preferably, the studied genes are integrated in said plasmids including their 3'- and 5'-end non-coding regions.

Individual S.cerevisiae genes may be studied using the above-described method, their sequences being totally or partially known. The complete genomic sequence of S.cerevisiae was made accessible to the public via the WWW (World Wide Web) on April 24, 1996.

Different possibilities exist to have access to the S.cerevisiae genomic DNA sequence via the WWW.
MIPS (Munich information Centre of Protein Sequence) Address http://speedy.mips.biochem.mpg.de/mips /yeast/
SGD (Saccharomyces Genome Database, Stanford) Address http://genome-www.stanford.edu/Saccharomyces
YPD(Yeast Protein Database, Cold Spring Harbor) Address http://www.proteome.com/YPDhome.html

The complete S.cerevisiae DNA sequence is also accessible via FTP (file transfer protocol) in Europe (e.g. at the address: ftp.mips.embnet.org) in the U.S.A. (address: genome-ftp.stanford.edu) or in Japan (address: ftp.nig.ac.jp).

3 essential genomic S.cerevisiae genes have been identified by this way, YNL256w, YBR102c and YIR012w.

The essential genes of S.cerevisiae are then used to identify corresponding functionally similar genes in other mycetes.

By functionally similar genes in other mycete species, is meant genes which have a function similar or identical to that of the identified essential genes of S.cerevisiae. Functionally similar genes in other mycetes may, but need not be homologous in sequence to the corresponding essential S.cerevisiae genes. Functionally similar genes in other mycetes may exhibit only moderate sequence homology at the nucleotide level to the corresponding essential S.cerevisiae genes. By moderate sequence homology it is meant in the present invention genes having a sequence identity, at the nucleotide level, of at least 50%, more preferably of at least 60% and most preferably of at least 70%.

In addition, functionally similar genes in other mycetes may, but need not encode proteins homologous in sequence to the proteins encoded by the essential S.cerevisiae genes. Functionally similar proteins in other mycetes may exhibit moderate protein sequence homology to the proteins encoded by the essential S.cerevisiae genes.

By moderate protein sequence homology is meant in the present invention proteins having a sequence identity, at the amino-acid level, of a least 40%, preferably of at least 50%, more preferably of at least 60% and most preferably of at least 70%.

Genes homologous in sequence may be isolated according to known methods, for example via homologous screening (Sambrook, J. et al. (1989) Molecular Cloning Cold Spring Harbor Laboratory Press, N.Y.) or via the PCR technique using specific primers from genomic libraries and/or cDNA libraries of the corresponding mycetes.

According to one embodiment, genes homologous in sequences are isolated from cDNA libraries. In order to find out functionally similar genes in other mycetes, mRNA is isolated from mycete species to be studied according to known methods (Sambrock et al. 1989) and cDNA is synthesized according to known methods (Sambrock et al. 1989; or cDNA synthesis kits, e.g. from STRATAGENE).

The prepared cDNA is directionally integrated in a suitable expression vector.

For example, synthesis of the first cDNA strand may be carried out in the presence of primers having appropriate restriction sites in order to allow a subsequent cloning in the proper orientation with respect to the expression vector promoter. As restriction sites, any known restriction site may be used. As a primer, for instance the following primer, 50 nucleotides long may be used:
5'-GAGAGAGAGAGAGAGAGAGAACTAGTXXXXXXTTTTTTTTTTTTTTTTTT-3'

The sequence (X)₆ represents an appropriate restriction site, for example for XhoI.

After two-strand synthesis, the cohesive ends of the double stranded cDNA are filled (blunt end) and the cDNA ends are then ligated using a suitable DNA adaptor sequence. The DNA adaptor sequence should contain a restriction site which should be different from the restriction site used in the primer for the synthesis of the first cDNA strand. The DNA adaptor may comprise for example complementary 9- or 13-mer oligonucleotides, whose ends represent the cohesive end of a restriction site. These ends may be for example a EcoRI-site:
5' XXXXXGGCACGAG 3'
3' XCCGTGCTC 5'

The single-stranded X in the adaptor sequence represent the cohesive end of a restriction site.

The cDNA provided with corresponding adaptor sequences is then cleaved using restriction endonuclease, whose recognition site was used in the primer for the synthesis of the first cDNA strand, for example XhoI. The cDNA thus obtained would have according to this example 3'-XhoI and 5'-EcoRI protruding ends and could be therefore directionally integrated into an expression vector cleaved with XhoI and EcoRI.

As expression vectors, among others, E. coli/S.cerevisiae shuttle vectors, i.e. vectors usable in E. coli as well as in S.cerevisiae are suitable. Such vectors may then be amplified for instance in E. coli. As expression vectors, those which are present in a high copy number as well as those present in a low copy number in S.cerevisiae cells can be used. For this purpose, for example vectors selected in the group consisting of pRS423 - pRS426 (pRS423, pRS424, pRS425, pRS426) and/or pRS313-pRS316 (pRS313, pRS314, pRS315, pRS316) (Sikorki, R.S. and Hieter, P. (1989) Genetics 122: 19-27; Christianson T. W. et al. (1992) Gene 110: 119-122) are suitable.

Expression vectors should contain appropriate S.cerevisiae promoters and terminators. In case they do not have these elements, the corresponding promoters and terminators are inserted in such a way that a subsequent incorporation of the generated cDNA remains possible. Particularly suitable are the promoters of S.cerevisiae genes MET25, PGK1, TPI1, TDH3, ADHI, URA3. One may use promoters of the wild-type gene in non modified form as well as promoters which were modified in such a way that certain activator sequences and/or repressor sequences were eliminated. As terminators, for example the terminators of the S.cerevisiae genes MET25, PGK1, TPI1, TDH3, ADHI, URA3 are suitable.

According to another embodiment, genes homologous in sequence are isolated from genomic libraries. Genomic DNA libraries from mycetes can be prepared according to procedures known (for example as described in Current Protocols in Molecular Biology, John Wiley and Sons, Inc). For example, genomic DNA from mycetes can be prepared using known methods for yeast cell lysis and isolation of genomic DNA (for example commercially available kits from Bio101, Inc). The genomic DNA can be partially digested using a restriction enzyme such as Sau3AI and the fragments are size-selected by agarose gel electrophoresis. DNA fragments having for example a size of 5-10kb are then purified by classical methods (as for example, using Gene Clean kit from Bio101) and inserted in a E.coli/yeast shuttle vector such as YEP24 (described e.g. by Sanglard D., Kuchler K., Ischer F., Pagani J-L., Monod M. and Bille J., Antimicrobial Agents and Chemotherapy, (1995) Vol.39 No11, P2378-2386) cut by a restriction enzyme giving compatible ends (for example BamHI for Sau3AI-cut genomic DNA). The resulting expression library can be amplified in E.coli. However any known method, appropriate for the preparation of a genomic library, can be used in the present invention.

In order to find the genes in the studied mycete species, which are functionally similar to essential genes of S.cerevisiae, one S.cerevisiae essential gene is placed under control of a regulated promoter, either as an integrative (1) or extrachromosomal (2) gene.
1. For the integration of a regulated promoter in the S.cerevisiae genome, one replaces the native promoter of the selected essential gene by the regulated promoter, for example by homologous recombination via PCR (Güldener et al. (1996). The homologous recombination via PCR can be carried out for example in the diploid S.cerevisiae strain CEN.PK2. The successfull integration into one chromosome can be checked in haploid cells following tetrad analysis.
   Using the tetrad analysis, one obtains four viable ascospores, wherein in two haploid segregates, the selected essential gene is placed under the control of the native promoter, while the essential gene in the two remaining segregates is placed under the control of the regulated promoter.
   The last mentioned haploid segregates are used for the transformation with the cDNA or the genomic DNA present in the recombinant vector.
2. Using the extrachromosomal variant, the selected essential S.cerevisiae gene is first inserted in a suitable expression vector, for example a E.coli/S.cerevisiae shuttle vector. For this purpose, the essential gene may be amplified via PCR from genomic S.cerevisiae DNA starting from the ATG initiation codon up to and including the termination codon. The primers used for this purpose may be constructed in such a way that they contain recognition sites for appropriate restriction enzymes, facilitating a subsequent insertion under control of a regulated promoter in an expression vector.

The recombinant expression vector with the plasmid copy of the essential S.cerevisiae gene under the control of a regulated promoter is subsequently used for the transcomplementation of the corresponding mutant allele. The corresponding mutant allele may be selected from the heterozygote-diploid mutant strains prepared by eliminating, partially or totally, by homologous recombination an essential mycete gene listed above and as described above.

The expression vector with the selected essential S.cerevisiae gene is transformed in the corresponding heterozygote-diploid mutant strain carrying instead of the selected essential S.cerevisiae gene, a selection marker gene. The transformants are isolated by selection based on the auxotrophic marker contained in the expression vector used. The thus transformed heterozygote-diploid mutant strains are submitted to a tetrad analysis. One obtains four viable segregates. By retracing the corresponding markers of the mutant allele and the expression vector, the transformed wild-type segregates may be distinguished from segregates which do not contain the genomic copy of the essential gene. Segregates, which do not contain the genomic copy of the selected essential gene, are designated as trans-complemented haploid mutant strains. They are subsequently used for transformation with cDNA or genomic DNA libraries from other mycete species present in appropriate vectors.

As regulated promoters, inducible or repressible promoters may be used. These promoters can consist of naturally and/or artificially disposed promoter sequences.

As regulated promoters, for example the promoters of GAL1 gene and the corresponding promoter derivatives, such as for example promoters, whose different UAS (upstream activation sequence) elements have been eliminated (GALS, GALL; Mumberg, J. et al. (1994) Nucleic Acids Research 22:5767-5768) may be used. As regulated promoters, promoters of gluconeogenic genes may also be used, such as e.g. FBP1, PCK1, ICL1 or parts therefrom, such as e.g. their activation sequence (UAS1 and/or UAS2) or repression sequence (URS, upstream repression sequence) (Niederacher et al. (1992), Curr. Genet. 22: 636-670; Proft et al. (1995) Mol. Gen. Gent. 246: 367-373; Schüller et al. (1992) EMBO J; 11: 107-114; Guarente et al. (1984) Cell 36: 503-511).

A S.cerevisiae mutant strain modified in this manner can be cultivated under growth conditions, in which the regulated promoter is active, so that the essential S.cerevisiae gene is expressed. The S.cerevisiae cells are then transformed with a representative quantity of the library containing the studied mycete species cDNA or genomic DNA. Transformants express additionally the protein whose coding sequence is present in the recombinant vector.

The method contemplates that the growth conditions may be modified in such a way as to inhibit the regulated promoter, under the control of which is the selected essential gene. Especially, growth conditions may be changed by replacing the growth medium. When for example the GAL1 promoter or a derivate thereof is used, one can replace the galactose-containing medium (induced state) by a glucose-containing medium (repressed state).

These modified conditions are lethal for the S.cerevisiae cells in which the recombinant vector does not carry the functionally similar genomic DNA or cDNA of the studied mycete species. On the contrary, the S.cerevisiae cells in which the recombinant vector expresses a functionally similar coding sequence of the studied mycete species, are viable, since in these cells the lethal metabolic defect is complemented by the protein encoded by the functionally similar gene.

The method contemplates that the recombinant vector (the plasmid) is isolated from the surviving transformants using known method (Strathern, J.N. and Higgins, D.R. (1991). Plasmids are recovered from yeast into Escherichia coli shuttle vectors in:Guthrie, C. and Fink, G.R. Methods in Enzymology, Volume 194. Guide to yeast genetic and molecular Biology. Academic Press, San Diego, 319-329) and the cDNA or genomic DNA is analyzed using DNA-analysis methods such as DNA sequencing. (Sanger et al. (1977), Proc. Natl. Acad. Sci. USA 74: 5463-5467)

Essential S.cerevisiae genes may thus be used for the identification of functionally similar genes and/or genes homologous in sequence in other mycetes, especially essential genes functionally similar and/or homologous in sequence in mycetes pathogenic to human, animal and plants. For this purpose for example mycetes of the classes phycomycetes or eumycetes may be used, in particular the subclasses basidiomycetes, ascomycetes, especially mehiascomycetales (yeast) and plectascales (mould fungus) and gymnascales (skin and hair fungus) or of the class of hyphomycetes, in particular the subclasses conidiosporales (skin fungus) and thallosporales (budding or gemmiparous fungus), among which particularly the species mucor, rhizopus, coccidioides, paracoccidioides (blastomyces brasiliensis), endomyces (blastomyces), aspergillus, penicilium (scopulariopsis), trichophyton (ctenomyces), epidermophton, microsporon, piedraia, hormodendron, phialophora, sporotrichon, cryptococcus, candida, geotrichum and trichosporon.

Of particular interest is the use of Candida Spp. especially Candida albicans, Candida glabrata, Aspergillus Spp., especially Aspergillus fumigatus, Coccidioides immitis, Cryptococcus neoformans, Histoplasma capsulatum, Blastomyces dermatitidis, Paracoccidioides brasiliens and Sporothrix schenckii.

Starting from the genes of S.cerevisiae, identified according to the above-described method, Applicants cloned corresponding essential genes from C.albicans i.e. CaNL256, CaYBR102 and CaIR012 by the following method.

First, oligonucleotide(s) is(are) selected in the sequence of the S.cerevisiae gene or a homologous C. albicans sequence in order to amplify the corresponding fragment of C.albicans. After cloning, the obtained fragment (exhibiting a sequence of about several hundred bp) is used as a probe for screening a C.albicans (genomic) DNA library. The screening may include the following steps: clones were spread on dishes, covered with filters where the DNA was crosslinked to the filters, filters are hybridized, the positive colonies are then detected. The selected clone(s) is (are) then sequenced.

The method contemplates that essential mycete genes are used to identify substances which may inhibit partially or totally the functional expression of these essential genes and/or the functional activity of the encoded proteins. Substances may be identified in this fashion, which inhibit mycetes growth and which can be used as antimycotics, for example in the preparation of drugs.

The present invention especially covers a method for screening such inhibiting substances wherein an essential gene from C.albicans selected from CaNL256, CaBR102 and CaIR012, or a functionally similar gene in another pathogenic mycete or the corresponding encoded protein is used as target.

By functionally similar genes in other pathogenic mycete species, as meant genes which have a function similar or identical to that of the identified essential genes of C.albicans. Functionally similar genes in other pathogenic mycetes may, but need not be homologous in sequence to the corresponding essential C.albicans genes. Functionally similar genes in other pathogenic mycetes may exhibit only moderate sequence homology at the nucleotide level to the corresponding essential C.albicans genes. By moderate sequence homology it is meant in the present invention genes having a sequence identity, at the nucleotide level, of at least 50%, more preferably of at least 60% and most preferably of at least 70%.

In addition, functionally similar genes in other pathogenic mycetes may, but need not encode proteins homologous in sequence to the proteins encoded by the essential C.albicans genes. Functionally similar proteins in other mycetes may exhibit moderate protein sequence homology to the proteins encoded by the essential C.albicans genes.

By moderate protein sequence homology is meant in the present invention proteins having a sequence identity, at the amino-acid level, of a least 40%, preferably of at least 50%, more preferably of at least 60% and most preferably of at least 70%.

A particular feature of this method is that essential mycete genes or the corresponding encoded proteins are used as targets for the screening of the substances. The method contemplates that essential C.albicans genes as well as functionally similar genes and/or genes homologous in sequence of other pathogenic mycetes or the corresponding encoded proteins may be used as targets.

According to one embodiment of the screening method of the invention, mycetes cells are provided, which contain the essential gene used as target, and those cells are incubated with the substance to be tested. By this way, the growth inhibitory effect of this substance with respect to the essential target gene is determined.

The mycetes cells which express the essential target gene to a different degree are used, and these cells are then incubated with the substance to be tested and the growth inhibitory effect of this substance is determined.

The method includes the use of two or more mycetes cells, or strains derived therefrom, which differ in that they express the essential target gene to a different degree.

For example, two, three, four, five, ten or more mycetes cells or the corresponding mycetes strains may be comparatively analysed with respect to the growth inhibitory effect of a substance used in a defined concentration. Through such expression degree series, antimycotic substances may be distinguished from cytotoxic or inactive substances.

A particular embodiment of the method includes the use of haploid mycetes cells/ strains for the screening, especially haploid S.cerevisiae cells/ strains.

The method contemplates the integration of the essential gene selected as a target in a suitable expression vector.

As expression vectors E.coli/S.cerevisiae shuttle vectors are for example suitable. Especially vectors differing in their copy number per cell may be used. Therefore, one may use vectors, which are present in the transformed S.cerevisiae cells in a high copy number, or one can also use those with a low copy number. One embodiment comprises the use of expression vectors which allow the integration of the target gene in the S.cerevisiae genome.

For example the vectors pRS423, pRS424,pRS425, pRS426, pRS313, pRS314, pRS315, pRS316, pRS303, pRS304, pRS305, pRS306 (Sikorki and Hieter, 1989; Christianson et al. 1992) are appropriate as expression vectors.

The vectors of the series pRS423 - pRS426 are present in a high copy number, about 50 - 100 copies/ cell. On the contrary, the vectors of the series pRS313 - pRS316 are present in a low copy number (1 - 2 copies / cell). When expression vectors from these two series are used, then the target gene is present as an extrachromosomal copy. Using the vector of the series pRS303 - pRS306 allows the integration of the target genes into the genome. Using these three different expression vector types allows a gradual expression of the studied functionally similar essential gene.

The method includes that the growth inhibitory effect of substances with respect to mycetes cells/strains is comparatively determined using expression vectors differing for instance in the copy number of the vector/cell.

Such cells may express the essential target gene to a different degree and may exhibit a graduated reaction with respect to the substance.

The method includes also, that a target gene expression of different strength is obtained in different mycetes cells (regulated overexpression) by insertion of the target gene in the expression vector between specific selected S.cerevisiae promoters and terminators. S.cerevisiae promoters which are constitutively expressed, but with different strength, are suitable. Examples for such promoters are native promoters of S.cerevisiae genes MET25, PGK1, TPI1, TDH3, ADH1, URA3, TRP1, as well as corresponding derivatives therefrom, for example promoter derivatives without specific activator and/or repressor sequences.

Regulated promoters are also appropriate for the graduated over-expression of the target gene. The native promoters of the GAL1 genes and/or corresponding derivates thereof, for example promoters, in which different UAS elements have been eliminated. (GALS, GALL; Mumberg et al. (1994) Nucleic Acids Research 22: 5767-5768) as well as promoters of gluconeogenic genes, for example the promoters FBP1, PCK1, ICL1, or parts thereof, for example their activator- (UAS1 or UAS2) or repressor- (URS) sequences are used in corresponding non activable and/or non repressible test promoters (Schüller et al. (1992) EMBO J. 11: 107-114) Guarente et al. (1984) Cell 36: 503-511; Niederacher et al. (1992) Curr. Genet. 22: 363-370; Proft et al. (1995) Mol. Gen. Genet. 246: 367-373).

In the expression vector terminator for example the terminator sequence of S.cerevisiae genes MET25, PGK1, TPI1, TDH3, ADHI, URA3 may be used.

The method includes that by the use of cleverly selected expression vector types and/or the preparation of suitable expression vectors, eventually using promoters of different strength and differently regulated promoters, a series of expression vectors may be constructed, all containing the same target gene, but differing in that they express the target gene to a different extent.

The method includes the transformation of the expression vector in haploid wild-type cells of S.cerevisiae. The thus obtained S.cerevisiae cells/strains are cultivated in liquid medium and incubated in the presence of different concentrations of the tested substance and the effect of this substance on the growth behaviour of the cells/strains expressing the target gene to a different degree is comparatively analysed. The method also includes that haploid S.cerevisiae cells/strains, transformed using the respective expression vector type without target gene, are used as a reference.

The method includes that the screening of the substances can be carried out in different media using regulated promoters, especially GAL1 promoter and its derivates (GALS and GALL), since the expression degree may be largely influenced by the choice of the respective medium. Thus, the expression degree of the GAL1 promoter decreases in the following fashion: 2 % galactose > 1 % galactose + 1 % glucose > 2 % glycerine > 2 % glucose.

The effect of the substances inhibiting the growth of wild-type cells of S.cerevisiae, may be partially or totally compensated by the overexpression of the functionally similar gene of another mycete species.

According to one embodiment, the method for screening antimycotic substances is carried out in vitro by contact of an essential or functionally similar gene or the corresponding encoded protein with the substance to be tested and determination of the effect of the substance on the target. Any in vitro test appropriate for determining the interaction of two molecules, such as a hybridization test or a functional test, can be used (e.g. enzymatic tests which are described in details in Bergmeyer H.U., Methods of Enzymatic Analysis, VCH Publishers). If the screening is carried out using the encoded protein as the target, then the corresponding essential gene is inserted by any suitable method known in the art, such as PCR amplification using a set of primers containing appropriate restriction sites, (Current Protocol in Molecular Biology, John Wiley and Sons, Inc) into an expression system, such as E. coli, Baculovirus, or yeast, and the expressed protein is then completely or partially purified by a method known in the art. Any purification method appropriate for the purification of expressed proteins, such as affinity chromatography can be used. If the target protein function is known, a functional test can then be carried out in which the effect of the antimycotic substance on the protein function is determined. If the protein function is unknown, substances which can interact with the target protein, e.g. which bind to the encoded protein, can be tested. In such a case a test such as protection of the target protein from enzymatic digestion by appropriate enzymes can be used.

According to one specific embodiment, the method for screening antimycotic substances corresponds to an enzymatic assay wherein the activity of dihydropteroate synthase (DHPS) and/or 7,8-dihydro-6-hydroxymethylpterin-pyrophosphokinase (HPPK) is determined; the enzymatic essay can be such as diclosed in "Bergmeyer H.U., Methods in Enzymatic analysis, VCH Publishers".

Dihydropteroate synthetase (DHPS) catalyses the condensation of 6-hydroxymethyl-7,8-dihydropterin pyrophosphate to para-aminobenzoic acid to form 7,8-dihydropteroate which corresponds to the second step in the three-step pathway leading from 6-hydroxymethyl-7,8-dihydropterin to 7,8-dihydrofolate. 7,8-dihydro-6-hydroxymethylpterin-pyrophosphokinase (HPPK) catalyzes the attachment of pyrophosphate to 6-hydroxymethyl-7,8-dihydropterin to form to 6-hydroxymethyl-7,8-dihydropterin pyrophosphate which corresponds to the first step in a three-step pathway leading to 7,8-dihydrofolate. All organisms require reduced folate cofactors for the synthesis of a variety of metabolites. Most microorganisms must synthesize folate de novo because they lack the active transport system of higher vertebrate cells which allows these organisms to use dietary folates. Enzymes involved in folate biosynthesis are therefore targets for a variety of antimicrobial agents. Consequently, these enzyme activities are essential to the microorganisms, and are absent in man.

The method also includes the identification of genes which are functionally similar and/or homologous in sequence to essential C.albicans genes from humans, animals or plants. The corresponding human, animal or plant genes may optionally be used as target genes in the method in order to test if antimycotic substances exhibit an effect on these target genes.

A particular advantage of the method is that in this way substances may be identified which efficiently inhibit mycetes growth and also the influence of these substances on corresponding functionally similar genes and/or genes homologous in sequence to essential C.albicans genes from human, animal or plants may be determined.

The method includes also the possibility to check the existence of functionally similar genes and/or human, animal or plant genes homologous in sequence to the corresponding essential mycete genes, for example by checking homology of the identified essential mycete genes or parts thereof with human, animal or plant sequence genes available in data banks. In this way, it is possible to select at an early stage from the identified essential mycete genes, depending on the aim, those for which no functionally similar gene and/or no human gene homologous in sequence exist, for example.

Thereby, the method offers a plurality of possibilities to identify selectively substances with antimycotic effects, with no harmful effect on human beings.

For example, it is possible to identify substances usable for the preparation of drugs for the treatment of mycosis or prophylaxis in immunodepression states. These substances may be used for example for the manufacture of drugs usable for the treatment of mycotic infections, which occur during diseases like Aids or Diabetes. Substances which may be used for the fabrication of fungicides, especially of fungicides which are harmless for humans and animals, can also be identified.

Furthermore, the method offers the possibility to identify antimycotic substances, which selectively inhibit growth of specific mycete species only.

The screening method is particularly advantageous inasmuch as it is sufficient to know whether the genes are essential, one does not need any additional information regarding the function of the essential genes or the function of the encoded proteins. In addition, it is particularly advantageous for the identification of functionally similar genes to essential S.cerevisiae gene, in other mycetes where the DNA sequence is not available for many of these genes.

According to another aspect the invention provides an antibody directed against the protein encoded by the CaNL256, CaBR102 or CaIR012 gene or a polypeptidic fragment thereof. The term "antibody" encompasses monoclonal and polyclonal antibodies. Said antibodies can be prepared by method well known in the art such as those disclosed in "Antibodies, a laboratory manual", Ed. Harbow and David Lane. Cold Spring Harbor Laboratory Eds., 1988.

According to another aspect the present invention provides a kit for the diagnosis of fungal infections comprising a gene selected from the group consisting of CaNL256, CaBR102 and CaIR012, a functionally similar gene thereof, a functional fragment thereof, the corresponding encoded protein, a functional polypeptide fragment thereof or an antiboby directed against the protein encoded by the CaNL256, CaBR102 or CaIR012 gene or a polypeptidic fragment thereof. Such kits can be prepared using any suitable method well known in the art.

### Examples

### Example 1 : CaNL256

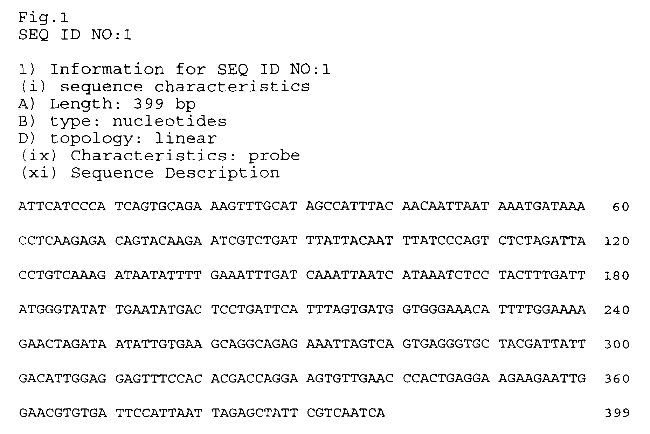

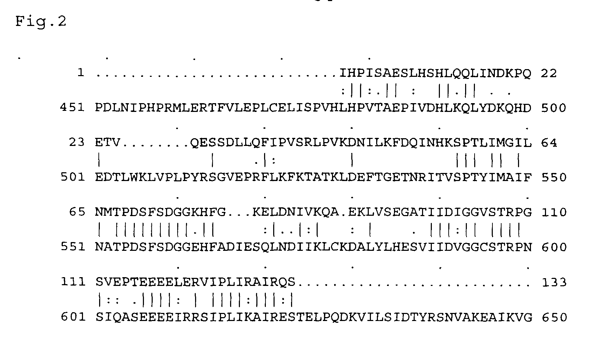

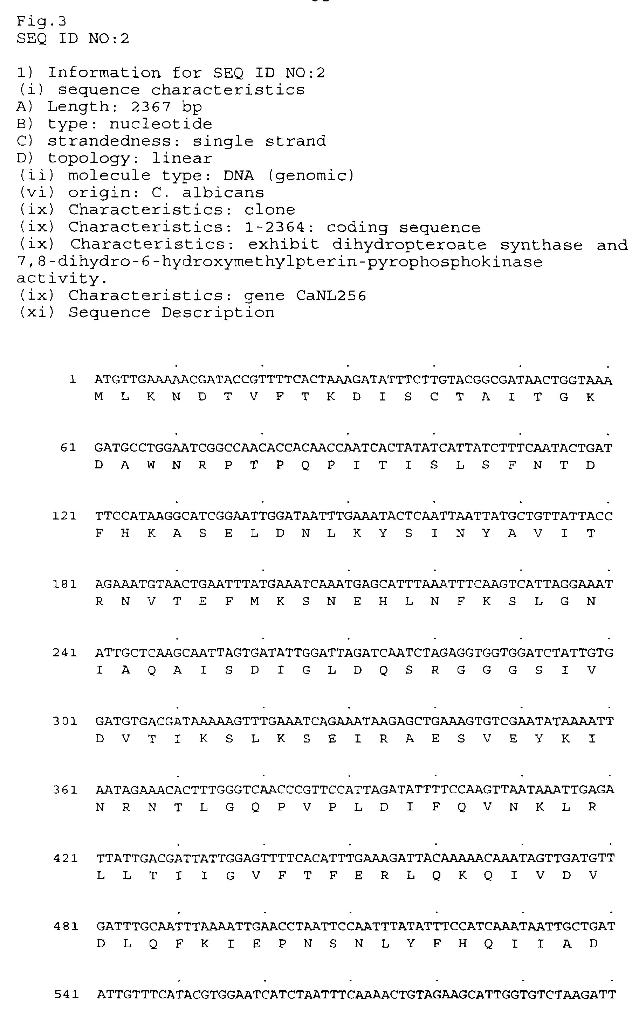

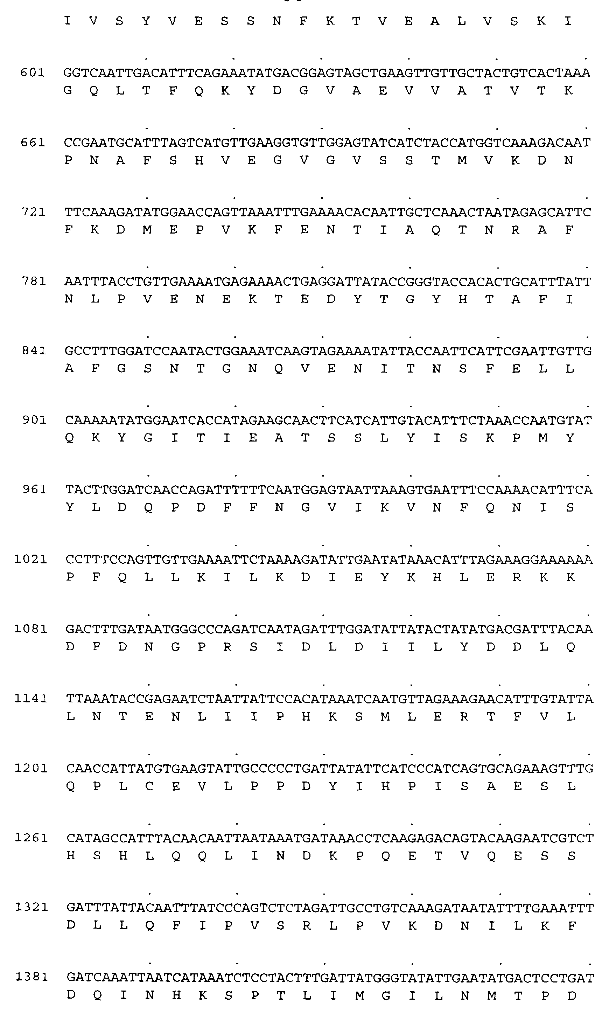

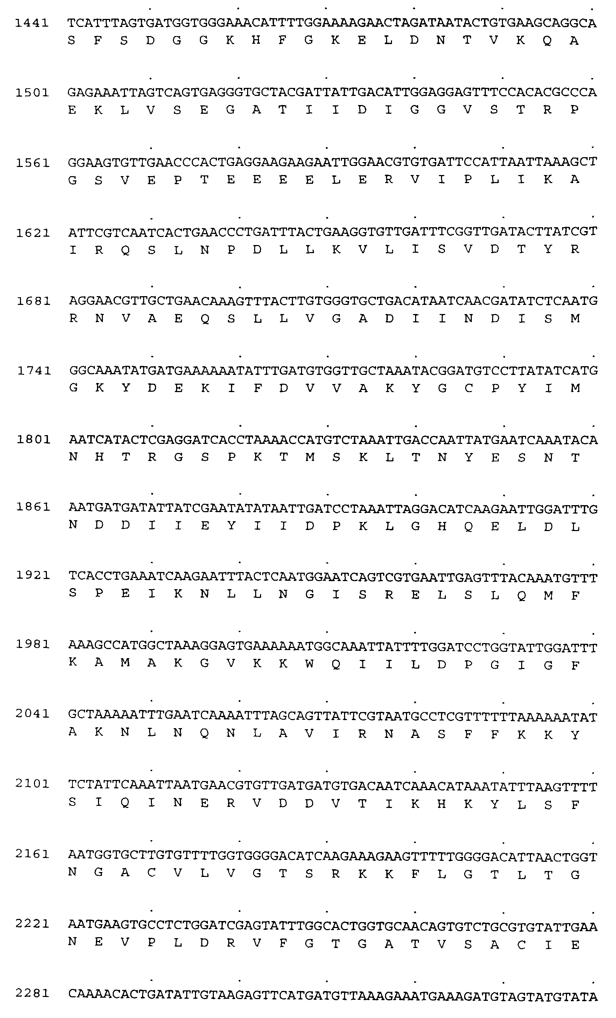

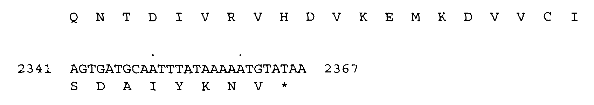

The Internet site of Standford (http://candida.standford.edu/) gives access to preliminary sequences of the genome of C. albicans. One of these sequences has homology with the YNL256 gene of S. cerevisiae. Two oligonucleotides were selected in this sequence (5'-ATTCATCCCATCAGTGCAGAAAG-3' and 5'-ATTGACCAATAGCTCTAATTAATG-3') in order to amplify the corresponding fragment of C. albicans. After cloning, we obtained a sequence of 399 bp close to the expected sequence (fig.1, SEQ ID NO:1). The deduced protein was compared with the one of YNL256, evidencing 53% similarity and 43% identity (fig.2). This fragment of 399 bp of C. albicans was used as a probe for screening a genomic library of C. albicans. The latter was prepared by partial digestion of genomic DNA of C. albicans by Sau3AI and cloning into the YEP24 vector at the BamHI site. The clones of the library were then spread at a density of 2000 clones per dish. Each dish was covered by a nitrocellulose filter which was then successively treated with: NaOH, 0.5M, 5 minutes; Tris, 1M, pH 7.7, 5 minutes; Tris, 0.5M, pH 7.7, Nacl, 1.25M, 5 minutes. After drying, the filters were kept for 2 hours at 80°C. Prehybridization and hybridization were carried out in a buffer of 40% formamide, 5xSSC, 20 mM Tris pH 7.7 1xDenhardt 0.1% SDS. The probe was labeled with 32P with the RediPrime kit and dCTP from Amersham UK. Hybridization took place over 17 hours at 42°C. The filters were then washed in 1x SSC, 0.1% SDS, three times for 5 minutes at room temperature and then twice for 30 minutes at 60°C, and were then submitted to autoradiography overnight. The colonies corresponding to the spots obtained were reisolated by re-spreading at low density followed by further hybridization. Three clones were thus obtained (out of 40,000), which were sequenced on an ABI 377 apparatus. The sequences were compiled using the ABI software and then analysed using the GCG software package. One of these three clones turned out to contain the complete coding sequence corresponding to the probe used; this gene was called CaNL256, whose sequence is represented Fig.3 (SEQ ID NO:2). CaNL256 has 52% of nucleotides identical to YNL256 of S. cerevisiae. The coding region is shorter at the N-terminus. For translation to amino acids, account was taken of the fact that, in C. albicans, the CTG codon is translated to Serine (there are 3 CTG codons in CaNL256). The deduced protein had 40% amino acids identical with YNL256 of S. cerevisiae and 41% with FAS (folic acid synthase) of Pneumocystis carinii. Investigation into the databases using the Blast software showed homology of two parts of the CaNL256 protein with, respectively, the bacterial enzymes Dihydropteroate Synthase (EC 2.5.1.15) (DHPS) of Haemophilus influenzae, Staphylococcus haemolyticus, Neisseria meningitidis, Streptococcus pneumoniae, Bacillus subtilis, Clostridium acetobutylicum, Escherichia coli, Mycobacterium leprae (P value less than e-28) and 7,8-dihydro-6-hydroxymethylpterin-pyrophosphokinase (EC 2.7.6.3) (HPPK) of Bacillus subtilis, Escherichia coli, Haemophilus influenzae, Streptococcus pneumoniae (P value less than e-20). The units characteristic of DHPS and HPPK are also found in CaNL256.

### Example 2 : CaBR102

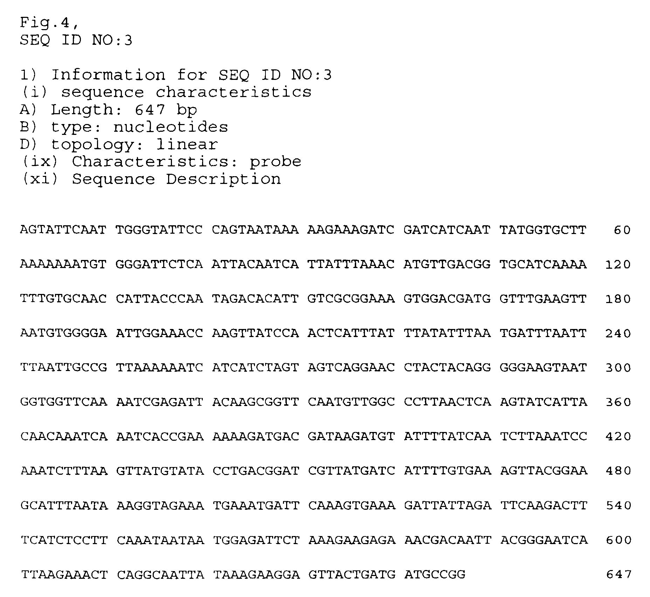

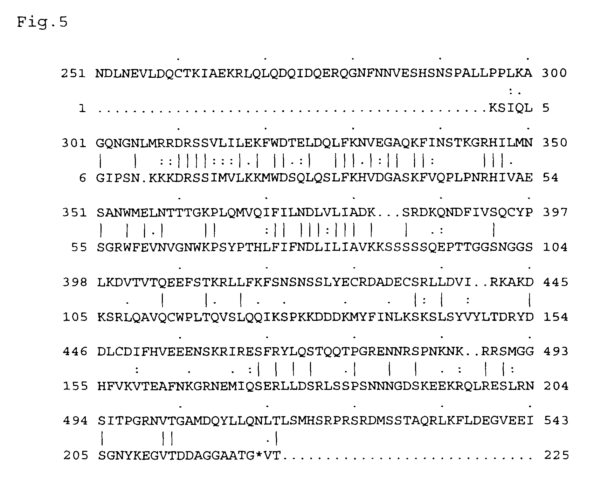

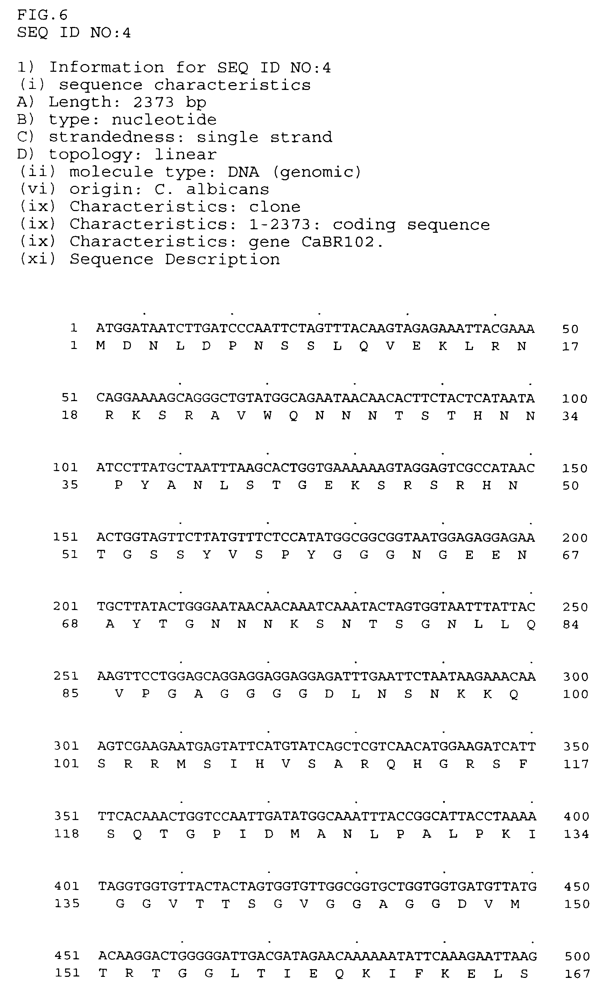

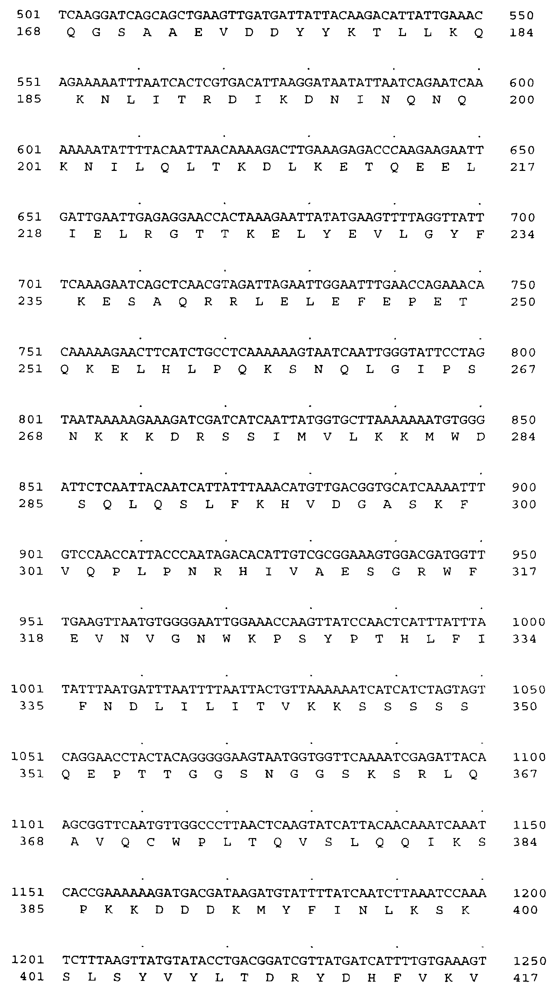

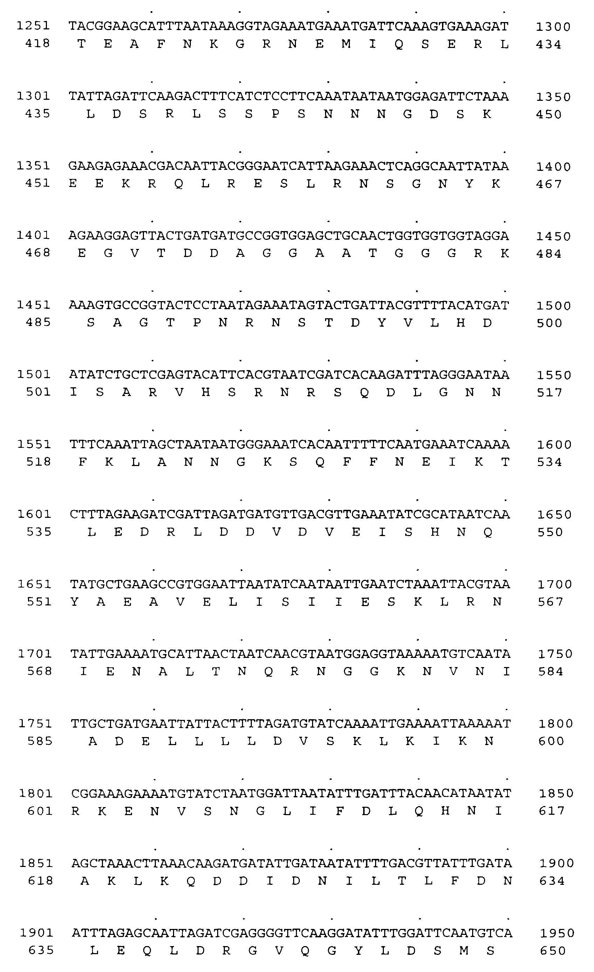

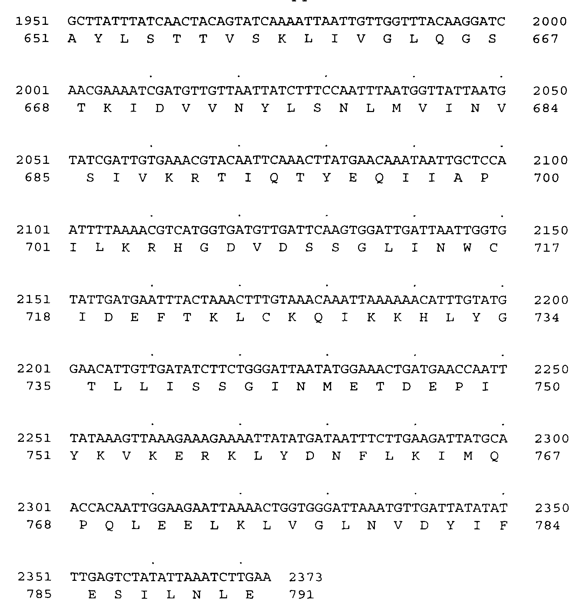

The Internet site of Stanford (http://candida.stanford.edu/) give access to preliminary sequences of the genome of C. albicans. One of these sequences has homology with the YBR102 gene of S.cerevisiae. Two oligonucleotides were selected in this sequence (5'-AGTATTCAATTGGGTATTCC-3' and 5'-CCGGCATCATCAGTAACTCC-3') in order to amplified the corresponding fragment of C. albicans. After cloning, we obtained a sequence of 647 bp close to the expected sequence (fig.4, SEQ ID NO:3). The deduced protein was compared with the one of YNL102, evidencing 35% similarity and 26% identity (fig.5). This fragment was amplified using Pfu polymerase (Stratagene). The PCR product was purified (High Pure PCR Product Purification Kit, Boehringer Mannheim) and used as a probe for screening a C. albicans genomic DNA library. The latter was prepared by partial digestion of C. albicans genomic DNA with SauIIIA and cloning into the YEP-24T_{RP}1 vector at the BamHI restriction site. 40,000 clones of the library were then spread at a density of 2000 clones per dish. Each dish was covered by a nitrocellulose filter (Membrane Hybond N⁺, Amersham) which was then successively treated with : 1.5 M NaCl/0.5 M NaOH, 5 minutes; 1.5 M NaCl/0.5 M Tris-HCl pH 7.2/1 mM EDTA, 3 minutes, twice; DNA was crosslinked to the filters (Amersham Life Science, ultra violet crosslinker). The probe (100 ng) was labelled with ³²P using the RediPrime kit and dCTP (Amersham Life Science). The filters were hybridized in a buffer containing 30% formamide, 5 x SSC, 5% Denhart's solution, 1% SDS, 100 µg /ml salmon sperm DNA and a probe concentration of 10⁶ cpm/ml at 42°C for 16 h. The membranes were then washed three times at room temperature in 2 x SSC/0.1% SDS for 5 minutes each and three times in 1 x SSC/0.1% SDS at 60°C for 20 minutes each. the filters were then exposed overnight to an X-ray film. The colonies corresponding to the positives clones were isolated and screened a second time by the same procedure. Two positives clones were finally obtained, which were sequenced on an ABI377 apparatus. the sequences were compiled using ABI software and then analysed using the GCG software package. The nucleotide sequences of these two clones were identical and contained the complete coding sequence corresponding to the probe used, this gene was called CaBR102, whose sequence is represented Fig.6 (SEQ ID NO:4). The translation of this nucleotide sequence was examined, account was taken of the fact that in C. albicans the CTG codon is translated to serine (there are 3 CTG codons in CaBR102). The deduced protein has 24% identity to S. cerevisiae gene YBR102.

### Example 3 : CaIR012

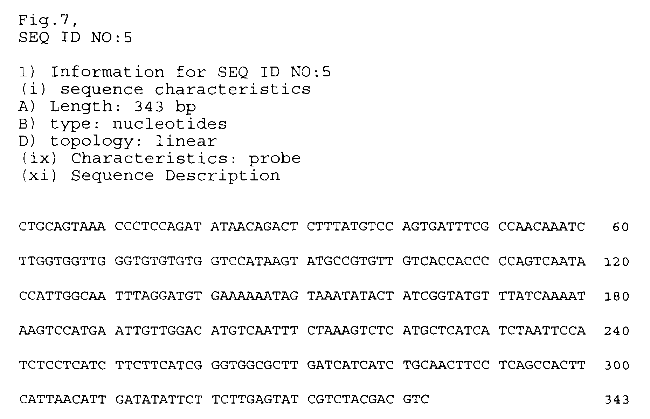

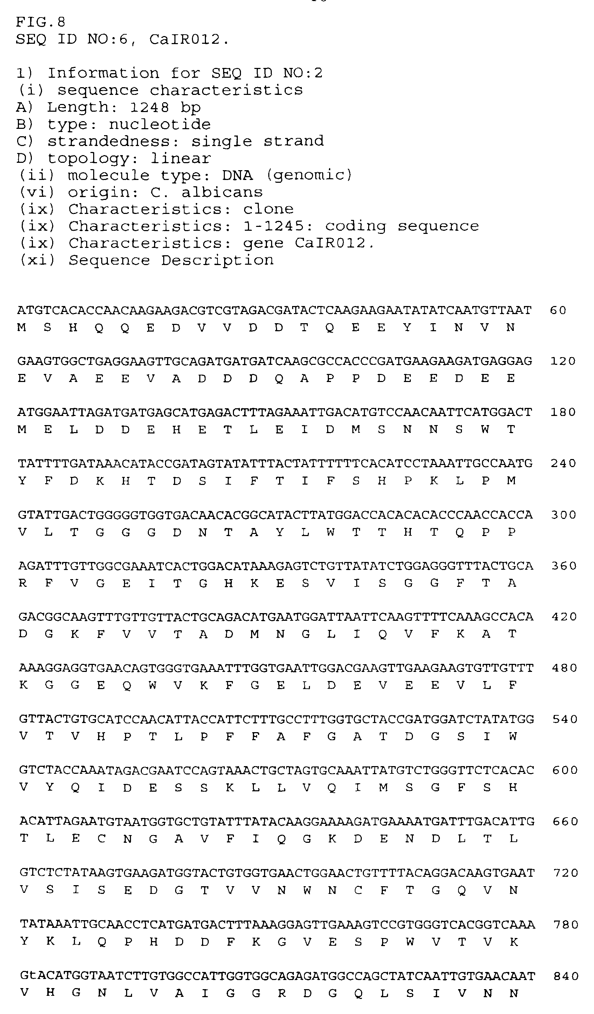

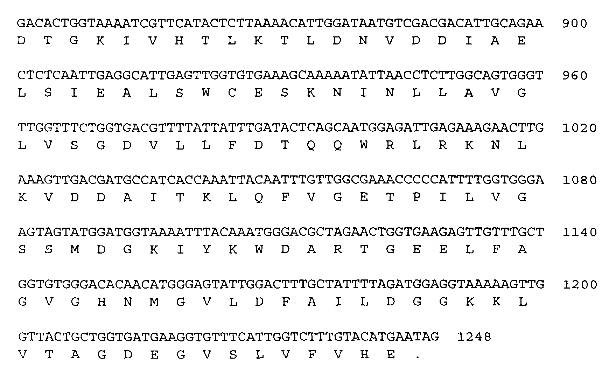

Chromosomal DNA from the C. albicans strain Caf2-1 was isolated using Yeast Cell Lysis prep Kit and Genome DNA Kit from BIO101. A 343 bp fragment from C. albicans genomic DNA (Fig.7, SEQ ID NO:5) was amplified with the oligonucleotide primers CaYIR012-5' (5'-GACGTCGTAGACGATACTCAAGAAG-3') and CaYIR012-3' (5'-CTGCAGTAAACCCTCCAGATATAACAG-3') by PowerScript DNA polymerase (PAN Systems GmbH) using the hot start technique. The PCR product was purified from the agarose gel and labeled with fluorescein (Gene image random prime labelling module, Amersham Life Science) according to the manufacturer's instructions. Plasmid DNA from E.coli was isolated using Qiagen columns as recommended by the manufacturer. Screening the λZAPII C. albicans cDNA library was performed following the manufacturer's instructions (Stratagene Ltd.). Nylon filters (Schleicher&Schuell) were lifted from LB-plates (150 mm) with 15000 pfu/plate, denatured 5 min in 1.5M NaCL, 0.5M NaOH, neutralized 3 min in 1.5M NaCl, 0.5M Tris-HCl pH8.0, washed 3 min in 0.2 M Tris-HCl pH 7.5, 2xSSC and DNA was crosslinked to the filters (Stratagene UV crosslinker). The filters were prehybridized 4 h at 60 °C and hybridized with the fluorescein-labeled DNA probe overnight at 60 °C. Detection was performed with Anti-fluorescein AP conjugate (Signal amplification module for the FluorImager, Amersham LIFE SCIENCE) and analysed after 20 h with a Fluorimager (Storm 860, Molecular Dynamics). Positive plaques were picked and incubated with 0.5 ml SM-buffer (100mM NaCl, 8 mM MgSO₄, 50 mM Tris-HCl pH7.5, 0,01% gelatin). The selected clones were diluted, titered with host cells XL1-Blue and screened and purified a second time by the same procedure. Finally, the pBluescript SK(-) phagemid containing the DNA insert of interest was rescued by the ExAssist Helper Phage system according to the Stratagene protocol. From a total of 75000 screened plaques, 3 positive clones were identified. pBluescript SK (-) phagemid DNA was isolated, sequenced with T3 and T7 primers and the sequences were extended with custom-synthesized oligonucleotide primers. Nucleotide sequence analyses were performed with the Gene Data software package (Gene Data AG, Easel Switzerland). Similarity searches with the Swissprot database were conducted with the BLAST program (Gish, Warren and David J. States (1993). Identification of protein coding regions by database similarity search. Nat. Genet. 3:266-72.). One of these three clones turned out to contain the complete coding sequence corresponding to the probe used; this gene was called CaIR1012, whose sequence is represented Fig.8 (SEQ ID NO:6).

## Claims

1. A method for the screening of antimycotic substances wherein an essential gene from mycetes or a functionally similar gene from another pathogenic mycete, or the corresponding encoded protein, is used as target and wherein the essential gene is selected from the group consisting in CaNL256, CaBR102 and CaIR012.

2. The method of claim 1 wherein mycete cells which express the essential gene, or a functionally similar mycete gene, to a different level are incubated with the substance to be tested and the growth inhibiting effect of the substance is determined.

3. The method of claim 1 wherein said target gene or the corresponding target encoded protein is contacted in vitro with the substance to be tested and the effect of the substance on the target is determined.

4. The method according to any one of claims 1-3 wherein the screened substances partially or totally inhibit the functional expression of the essential genes or the functional activity of the encoded proteins.

5. The method according to any one of claims 1-4 wherein the screened substances partially or totally inhibit the activity of dihydropteroate synthase (DHPS) and/or 7,8-dihydro-6-hydroxymethylpterin-pyrophosphokinase (HPPK).

6. The method according to any one of claims 1-5 wherein the mycete species are selected from the group comprising Basidiomycetes, Ascomycetes and Hyphomycetes.

7. The method according to any one of claims 1-6, wherein said functionally similar genes are essential genes from Candida Spp, or Aspergillus Spp.

8. The method according to claim 7, wherein said functionally similar genes are essential genes from Candida albicans, or Aspergillus fumigatus.

9. The method according to any one of claims 1-8 wherein the functionally similar gene has a sequence identity, at the nucleotide level, with the corresponding essential gene of at least 50%, preferably of at least 60%, and most preferably of at least 70%.

10. The method according to any one of claims 1-8 wherein the functionally similar gene encodes a protein having a sequence identity, at the amino-acid level, with the corresponding essential gene encoded protein of at least 40%, preferably of at least 50%, more preferably of at least 60% and most preferably of at least 70%.

11. Gene CaNL256 having the sequence corresponding to SEQ ID NO:2, a functionally similar gene or a functional fragment thereof.

12. Gene CaBR102 having the sequence corresponding to SEQ ID NO:4, a functionally similar gene or a functional fragment thereof.

13. Gene CaIR012 having the sequence corresponding to SEQ ID NO:6, a functionally similar gene or a functional fragment thereof.

14. A gene according to claim 11, 12 or 13, wherein the functionally similar gene has a sequence identity, at the nucleotide level, of at least 50%, preferably of at least 60%, and most preferably of at least 70%.

15. A gene according to claim 11, 12 or 13, wherein the functionally similar gene has a sequence identity, at the amino-acid level, with the encoded protein, of at least 40%, preferably of at least 50%, more preferably of at least 60% and most preferably of at least 70%.

16. A protein encoded by the gene of claim 11, 12 or 13, or a functional polypeptide fragment thereof.

17. A plasmid containing the gene of claim 11, 12 or 13.

18. A plasmid deposited at the CNCM with the accession number I-2065.

19. A plasmid deposited at the CNCM with the accession number I-2063.

20. A plasmid deposited at the CNCM with the accession number I-2064.

21. An antiboby directed against the protein of claim 16 or a functional polypeptide fragment thereof.

22. A kit for diagnosis of fungal infections comprising a gene selected from the group consisting of CaNL256, CaBR102 and CaIR012, a functionally similar gene thereof, a functional fragment thereof, the corresponding encoded protein or a functional polypeptidic fragment thereof, or an antiboby directed against the protein encoded by the gene selected from the group consisting of CaNL256, CaBR102 and CaIR012, or a polypeptidic fragment thereof.


SEQUENCE LISTING

<110> HOECHST MARION ROUSSEL

<120> METHOD FOR SCREENING ANTIMYCOTIC SUBSTANCES USING
      ESSENTIAL GENES FROM C.ALBICANS, AND SAID GENES

<130> 15801 EP RUU

<140> 98402255.8
<141> 1998-09-11

<160> 9

<170> PatentIn Ver. 2.1

<210> 1
<211> 399
<212> DNA
<213> Artificial Sequence

<220>
<223> Description of Artificial Sequence: probe

<400> 1
attcatccca tcagtgcaga aagtttgcat agccatttac aacaattaat aaatgataaa 60

cctcaagaga cagtacaaga atcgtctgat ttattacaat ttatcccagt ctctagatta 120

cctgtcaaag ataatatttt gaaatttgat caaattaatc ataaatctcc tactttgatt 180

atgggtatat tgaatatgac tcctgattca tttagtgatg gtgggaaaca ttttggaaaa 240

gaactagata atattgtgaa gcaggcagag aaattagtca gtgagggtgc tacgattatt 300

gacattggag gagtttccac acgaccagga agtgttgaac ccactgagga agaagaattg 360

gaacgtgtga ttccattaat tagagctatt cgtcaatca 399

<210> 2
<211> 343
<212> DNA
<213> Artificial Sequence

<220>
<223> Description of Artificial Sequence: probe

<400> 2
ctgcagtaaa ccctccagat ataacagact ctttatgtcc agtgatttcg ccaacaaatc 60

ttggtggttg ggtgtgtgtg gtccataagt atgccgtgtt gtcaccaccc ccagtcaata 120

ccattggcaa tttaggatgt gaaaaaatag taaatatact atcggtatgt ttatcaaaat 180

aagtccatga attgttggac atgtcaattt ctaaagtctc atgctcatca tctaattcca 240

tctcctcatc ttcttcatcg ggtggcgctt gatcatcatc tgcaacttcc tcagccactt 300

cattaacatt gatatattct tcttgagtat cgtctacgac gtc 343

<210> 3
<211> 2367
<212> DNA

<213> Candida albicans

<220>
<221> CDS
<222> (1)..(2364)

<220>
<221> gene
<222> (1)..(2364)
<223> gene CaNL256 exhibit dihydropteroate synthase and
      7,8-dihydro-6-hydroxymethylpterin-pyrophosphokinas
      e
      genomic DNA

<400> 3
atg ttg aaa aac gat acc gtt ttc act aaa gat att tct tgt acg gcg      48
Met Leu Lys Asn Asp Thr Val Phe Thr Lys Asp Ile Ser Cys Thr Ala
1               5                   10                  15

ata act ggt aaa gat gcc tgg aat cgg cca aca cca caa cca atc act      96
Ile Thr Gly Lys Asp Ala Trp Asn Arg Pro Thr Pro Gln Pro Ile Thr
            20                  25                  30

ata tca tta tct ttc aat act gat ttc cat aag gca tcg gaa ttg gat      144
Ile Ser Leu Ser Phe Asn Thr Asp Phe His Lys Ala Ser Glu Leu Asp
        35                  40                  45

aat ttg aaa tac tca att aat tat gct gtt att acc aga aat gta act      192
Asn Leu Lys Tyr Ser Ile Asn Tyr Ala Val Ile Thr Arg Asn Val Thr
    50                  55                  60

gaa ttt atg aaa tca aat gag cat tta aat ttc aag tca tta gga aat      240
Glu Phe Met Lys Ser Asn Glu His Leu Asn Phe Lys Ser Leu Gly Asn
65                  70                  75                  80

att gct caa gca att agt gat att gga tta gat caa tct aga ggt ggt      288
Ile Ala Gln Ala Ile Ser Asp Ile Gly Leu Asp Gln Ser Arg Gly Gly
                85                  90                  95

gga tct att gtg gat gtg acg ata aaa agt ttg aaa tca gaa ata aga      336
Gly Ser Ile Val Asp Val Thr Ile Lys Ser Leu Lys Ser Glu Ile Arg
            100                 105                 110

gct gaa agt gtc gaa tat aaa att aat aga aac act ttg ggt caa ccc      384
Ala Glu Ser Val Glu Tyr Lys Ile Asn Arg Asn Thr Leu Gly Gln Pro
        115                 120                 125

gtt cca tta gat att ttc caa gtt aat aaa ttg aga tta ttg acg att      432
Val Pro Leu Asp Ile Phe Gln Val Asn Lys Leu Arg Leu Leu Thr Ile
    130                 135                 140

att gga gtt ttc aca ttt gaa aga tta caa aaa caa ata gtt gat gtt      480
Ile Gly Val Phe Thr Phe Glu Arg Leu Gln Lys Gln Ile Val Asp Val
145                 150                 155                 160

gat ttg caa ttt aaa att gaa cct aat tcc aat tta tat ttc cat caa      528
Asp Leu Gln Phe Lys Ile Glu Pro Asn Ser Asn Leu Tyr Phe His Gln
                165                 170                 175

ata att gct gat att gtt tca tac gtg gaa tca tct aat ttc aaa act      576
Ile Ile Ala Asp Ile Val Ser Tyr Val Glu Ser Ser Asn Phe Lys Thr
            180                 185                 190

gta gaa gca ttg gtg tct aag att ggt caa ttg aca ttt cag aaa tat      624
Val Glu Ala Leu Val Ser Lys Ile Gly Gln Leu Thr Phe Gln Lys Tyr

195                 200                 205

gac gga gta gct gaa gtt gtt gct act gtc act aaa ccg aat gca ttt     672
Asp Gly Val Ala Glu Val Val Ala Thr Val Thr Lys Pro Asn Ala Phe
    210                 215                 220

agt cat gtt gaa ggt gtt gga gta tca tct acc atg gtc aaa gac aat     720
Ser His Val Glu Gly Val Gly Val Ser Ser Thr Met Val Lys Asp Asn
225                 230                 235                 240

ttc aaa gat atg gaa cca gtt aaa ttt gaa aac aca att gct caa act     768
Phe Lys Asp Met Glu Pro Val Lys Phe Glu Asn Thr Ile Ala Gln Thr
                245                 250                 255

aat aga gca ttc aat tta cct gtt gaa aat gag aaa act gag gat tat     816
Asn Arg Ala Phe Asn Leu Pro Val Glu Asn Glu Lys Thr Glu Asp Tyr
            260                 265                 270

acc ggg tac cac act gca ttt att gcc ttt gga tcc aat act gga aat     864
Thr Gly Tyr His Thr Ala Phe Ile Ala Phe Gly Ser Asn Thr Gly Asn
        275                 280                 285

caa gta gaa aat att acc aat tca ttc gaa ttg ttg caa aaa tat gga     912
Gln Val Glu Asn Ile Thr Asn Ser Phe Glu Leu Leu Gln Lys Tyr Gly
    290                 295                 300

atc acc ata gaa gca act tca tca ttg tac att tct aaa cca atg tat     960
Ile Thr Ile Glu Ala Thr Ser Ser Leu Tyr Ile Ser Lys Pro Met Tyr
305                 310                 315                 320

tac ttg gat caa cca gat ttt ttc aat gga gta att aaa gtg aat ttc     1008
Tyr Leu Asp Gln Pro Asp Phe Phe Asn Gly Val Ile Lys Val Asn Phe
                325                 330                 335

caa aac att tca cct ttc cag ttg ttg aaa att cta aaa gat att gaa     1056
Gln Asn Ile Ser Pro Phe Gln Leu Leu Lys Ile Leu Lys Asp Ile Glu
            340                 345                 350

tat aaa cat tta gaa agg aaa aaa gac ttt gat aat ggg ccc aga tca     1104
Tyr Lys His Leu Glu Arg Lys Lys Asp Phe Asp Asn Gly Pro Arg Ser
        355                 360                 365

ata gat ttg gat att ata cta tat gac gat tta caa tta aat acc gag     1152
Ile Asp Leu Asp Ile Ile Leu Tyr Asp Asp Leu Gln Leu Asn Thr Glu
    370                 375                 380

aat cta att att cca cat aaa tca atg tta gaa aga aca ttt gta tta     1200
Asn Leu Ile Ile Pro His Lys Ser Met Leu Glu Arg Thr Phe Val Leu
385                 390                 395                 400

caa cca tta tgt gaa gta ttg ccc cct gat tat att cat ccc atc agt     1248
Gln Pro Leu Cys Glu Val Leu Pro Pro Asp Tyr Ile His Pro Ile Ser
                405                 410                 415

gca gaa agt ttg cat agc cat tta caa caa tta ata aat gat aaa cct     1296
Ala Glu Ser Leu His Ser His Leu Gln Gln Leu Ile Asn Asp Lys Pro
            420                 425                 430

caa gag aca gta caa gaa tcg tct gat tta tta caa ttt atc cca gtc     1344
Gln Glu Thr Val Gln Glu Ser Ser Asp Leu Leu Gln Phe Ile Pro Val
        435                 440                 445

tct aga ttg cct gtc aaa gat aat att ttg aaa ttt gat caa att aat     1392
Ser Arg Leu Pro Val Lys Asp Asn Ile Leu Lys Phe Asp Gln Ile Asn
    450                 455                 460

cat aaa tct cct act ttg att atg ggt ata ttg aat atg act cct gat     1440
His Lys Ser Pro Thr Leu Ile Met Gly Ile Leu Asn Met Thr Pro Asp
465                 470                 475                 480

tca ttt agt gat ggt ggg aaa cat ttt gga aaa gaa cta gat aat act     1488
Ser Phe Ser Asp Gly Gly Lys His Phe Gly Lys Glu Leu Asp Asn Thr
                485                 490                 495

gtg aag cag gca gag aaa tta gtc agt gag ggt gct acg att att gac     1536
Val Lys Gln Ala Glu Lys Leu Val Ser Glu Gly Ala Thr Ile Ile Asp
            500                 505                 510

att gga gga gtt tcc aca cgc cca gga agt gtt gaa ccc act gag gaa     1584
Ile Gly Gly Val Ser Thr Arg Pro Gly Ser Val Glu Pro Thr Glu Glu
        515                 520                 525

gaa gaa ttg gaa cgt gtg att cca tta att aaa gct att cgt caa tca     1632
Glu Glu Leu Glu Arg Val Ile Pro Leu Ile Lys Ala Ile Arg Gln Ser
    530                 535                 540

ctg aac cct gat tta ctg aag gtg ttg att tcg gtt gat act tat cgt     1680
Leu Asn Pro Asp Leu Leu Lys Val Leu Ile Ser Val Asp Thr Tyr Arg
545                 550                 555                 560

agg aac gtt gct gaa caa agt tta ctt gtg ggt gct gac ata atc aac     1728
Arg Asn Val Ala Glu Gln Ser Leu Leu Val Gly Ala Asp Ile Ile Asn
                565                 570                 575

gat atc tca atg ggc aaa tat gat gaa aaa ata ttt gat gtg gtt gct     1776
Asp Ile Ser Met Gly Lys Tyr Asp Glu Lys Ile Phe Asp Val Val Ala
            580                 585                 590

aaa tac gga tgt cct tat atc atg aat cat act cga gga tca cct aaa     1824
Lys Tyr Gly Cys Pro Tyr Ile Met Asn His Thr Arg Gly Ser Pro Lys
        595                 600                 605

acc atg tct aaa ttg acc aat tat gaa tca aat aca aat gat gat att     1872
Thr Met Ser Lys Leu Thr Asn Tyr Glu Ser Asn Thr Asn Asp Asp Ile
    610                 615                 620

atc gaa tat ata att gat cct aaa tta gga cat caa gaa ttg gat ttg     1920
Ile Glu Tyr Ile Ile Asp Pro Lys Leu Gly His Gln Glu Leu Asp Leu
625                 630                 635                 640

tca cct gaa atc aag aat tta ctc aat gga atc agt cgt gaa ttg agt     1968
Ser Pro Glu Ile Lys Asn Leu Leu Asn Gly Ile Ser Arg Glu Leu Ser
                645                 650                 655

tta caa atg ttt aaa gcc atg gct aaa gga gtg aaa aaa tgg caa att     2016
Leu Gln Met Phe Lys Ala Met Ala Lys Gly Val Lys Lys Trp Gln Ile
            660                 665                 670

att ttg gat cct ggt att gga ttt gct aaa aat ttg aat caa aat tta     2064
Ile Leu Asp Pro Gly Ile Gly Phe Ala Lys Asn Leu Asn Gln Asn Leu
        675                 680                 685

gca gtt att cgt aat gcc tcg ttt ttt aaa aaa tat tct att caa att     2112
Ala Val Ile Arg Asn Ala Ser Phe Phe Lys Lys Tyr Ser Ile Gln Ile
    690                 695                 700

aat gaa cgt gtt gat gat gtg aca atc aaa cat aaa tat tta agt ttt     2160
Asn Glu Arg Val Asp Asp Val Thr Ile Lys His Lys Tyr Leu Ser Phe
705                 710                 715                 720

aat ggt gct tgt gtt ttg gtg ggg aca tca aga aag aag ttt ttg ggg     2208

Asn Gly Ala Cys Val Leu Val Gly Thr Ser Arg Lys Lys Phe Leu Gly
                725                 730                 735

aca tta act ggt aat gaa gtg cct ctg gat cga gta ttt ggc act ggt    2256
Thr Leu Thr Gly Asn Glu Val Pro Leu Asp Arg Val Phe Gly Thr Gly
            740                 745                 750

gca aca gtg tct gcg tgt att gaa caa aac act gat att gta aga gtt    2304
Ala Thr Val Ser Ala Cys Ile Glu Gln Asn Thr Asp Ile Val Arg Val
        755                 760                 765

cat gat gtt aaa gaa atg aaa gat gta gta tgt ata agt gat gca att    2352
His Asp Val Lys Glu Met Lys Asp Val Val Cys Ile Ser Asp Ala Ile
    770                 775                 780

tat aaa aat gta taa                                                2367
Tyr Lys Asn Val
785


<210> 4
<211> 788
<212> PRT
<213> Candida albicans

<400> 4
Met Leu Lys Asn Asp Thr Val Phe Thr Lys Asp Ile Ser Cys Thr Ala
  1               5                  10                  15

Ile Thr Gly Lys Asp Ala Trp Asn Arg Pro Thr Pro Gln Pro Ile Thr
             20                  25                  30

Ile Ser Leu Ser Phe Asn Thr Asp Phe His Lys Ala Ser Glu Leu Asp
         35                  40                  45

Asn Leu Lys Tyr Ser Ile Asn Tyr Ala Val Ile Thr Arg Asn Val Thr
     50                  55                  60

Glu Phe Met Lys Ser Asn Glu His Leu Asn Phe Lys Ser Leu Gly Asn
 65                  70                  75                  80

Ile Ala Gln Ala Ile Ser Asp Ile Gly Leu Asp Gln Ser Arg Gly Gly
                 85                  90                  95

Gly Ser Ile Val Asp Val Thr Ile Lys Ser Leu Lys Ser Glu Ile Arg
            100                 105                 110

Ala Glu Ser Val Glu Tyr Lys Ile Asn Arg Asn Thr Leu Gly Gln Pro
        115                 120                 125

Val Pro Leu Asp Ile Phe Gln Val Asn Lys Leu Arg Leu Leu Thr Ile
    130                 135                 140

Ile Gly Val Phe Thr Phe Glu Arg Leu Gln Lys Gln Ile Val Asp Val
145                 150                 155                 160

Asp Leu Gln Phe Lys Ile Glu Pro Asn Ser Asn Leu Tyr Phe His Gln
                165                 170                 175

Ile Ile Ala Asp Ile Val Ser Tyr Val Glu Ser Ser Asn Phe Lys Thr
            180                 185                 190

Val Glu Ala Leu Val Ser Lys Ile Gly Gln Leu Thr Phe Gln Lys Tyr
        195                 200                 205

Asp Gly Val Ala Glu Val Val Ala Thr Val Thr Lys Pro Asn Ala Phe
    210                 215                 220

Ser His Val Glu Gly Val Gly Val Ser Ser Thr Met Val Lys Asp Asn
225                 230                 235                 240

Phe Lys Asp Met Glu Pro Val Lys Phe Glu Asn Thr Ile Ala Gln Thr
                245                 250                 255

Asn Arg Ala Phe Asn Leu Pro Val Glu Asn Glu Lys Thr Glu Asp Tyr
            260                 265                 270

Thr Gly Tyr His Thr Ala Phe Ile Ala Phe Gly Ser Asn Thr Gly Asn
        275                 280                 285

Gln Val Glu Asn Ile Thr Asn Ser Phe Glu Leu Leu Gln Lys Tyr Gly
    290                 295                 300

Ile Thr Ile Glu Ala Thr Ser Ser Leu Tyr Ile Ser Lys Pro Met Tyr
305                 310                 315                 320

Tyr Leu Asp Gln Pro Asp Phe Phe Asn Gly Val Ile Lys Val Asn Phe
                325                 330                 335

Gln Asn Ile Ser Pro Phe Gln Leu Leu Lys Ile Leu Lys Asp Ile Glu
            340                 345                 350

Tyr Lys His Leu Glu Arg Lys Lys Asp Phe Asp Asn Gly Pro Arg Ser
        355                 360                 365

Ile Asp Leu Asp Ile Ile Leu Tyr Asp Asp Leu Gln Leu Asn Thr Glu
    370                 375                 380

Asn Leu Ile Ile Pro His Lys Ser Met Leu Glu Arg Thr Phe Val Leu
385                 390                 395                 400

Gln Pro Leu Cys Glu Val Leu Pro Pro Asp Tyr Ile His Pro Ile Ser
                405                 410                 415

Ala Glu Ser Leu His Ser His Leu Gln Gln Leu Ile Asn Asp Lys Pro
            420                 425                 430

Gln Glu Thr Val Gln Glu Ser Ser Asp Leu Leu Gln Phe Ile Pro Val
        435                 440                 445

Ser Arg Leu Pro Val Lys Asp Asn Ile Leu Lys Phe Asp Gln Ile Asn
    450                 455                 460

His Lys Ser Pro Thr Leu Ile Met Gly Ile Leu Asn Met Thr Pro Asp
465                 470                 475                 480

Ser Phe Ser Asp Gly Gly Lys His Phe Gly Lys Glu Leu Asp Asn Thr
                485                 490                 495

Val Lys Gln Ala Glu Lys Leu Val Ser Glu Gly Ala Thr Ile Ile Asp
            500                 505                 510

Ile Gly Gly Val Ser Thr Arg Pro Gly Ser Val Glu Pro Thr Glu Glu
        515                 520                 525

Glu Glu Leu Glu Arg Val Ile Pro Leu Ile Lys Ala Ile Arg Gln Ser
    530                 535                 540

Leu Asn Pro Asp Leu Leu Lys Val Leu Ile Ser Val Asp Thr Tyr Arg
545                 550                 555                 560

Arg Asn Val Ala Glu Gln Ser Leu Leu Val Gly Ala Asp Ile Ile Asn
                565                 570                 575

Asp Ile Ser Met Gly Lys Tyr Asp Glu Lys Ile Phe Asp Val Val Ala
            580                 585                 590

Lys Tyr Gly Cys Pro Tyr Ile Met Asn His Thr Arg Gly Ser Pro Lys
        595                 600                 605

Thr Met Ser Lys Leu Thr Asn Tyr Glu Ser Asn Thr Asn Asp Asp Ile
    610                 615                 620

Ile Glu Tyr Ile Ile Asp Pro Lys Leu Gly His Gln Glu Leu Asp Leu
625                 630                 635                 640

Ser Pro Glu Ile Lys Asn Leu Leu Asn Gly Ile Ser Arg Glu Leu Ser
                645                 650                 655

Leu Gln Met Phe Lys Ala Met Ala Lys Gly Val Lys Lys Trp Gln Ile
            660                 665                 670

Ile Leu Asp Pro Gly Ile Gly Phe Ala Lys Asn Leu Asn Gln Asn Leu
        675                 680                 685

Ala Val Ile Arg Asn Ala Ser Phe Phe Lys Lys Tyr Ser Ile Gln Ile
    690                 695                 700

Asn Glu Arg Val Asp Asp Val Thr Ile Lys His Lys Tyr Leu Ser Phe
705                 710                 715                 720

Asn Gly Ala Cys Val Leu Val Gly Thr Ser Arg Lys Lys Phe Leu Gly
                725                 730                 735

Thr Leu Thr Gly Asn Glu Val Pro Leu Asp Arg Val Phe Gly Thr Gly
            740                 745                 750

Ala Thr Val Ser Ala Cys Ile Glu Gln Asn Thr Asp Ile Val Arg Val
        755                 760                 765

His Asp Val Lys Glu Met Lys Asp Val Val Cys Ile Ser Asp Ala Ile
    770                 775                 780

Tyr Lys Asn Val
785


<210> 5
<211> 2373
<212> DNA
<213> Candida albicans

<220>
<221> CDS
<222> (1)..(2373)

<220>
<221> gene
<222> (1)..(2373)
<223> gene CaBR102

<400> 5
atg gat aat ctt gat ccc aat tct agt tta caa gta gag aaa tta cga      48
Met Asp Asn Leu Asp Pro Asn Ser Ser Leu Gln Val Glu Lys Leu Arg
1               5                   10                  15

aac agg aaa agc agg gct gta tgg cag aat aac aac act tct act cat    96
Asn Arg Lys Ser Arg Ala Val Trp Gln Asn Asn Asn Thr Ser Thr His
            20                  25                  30

aat aat cct tat gct aat tta agc act ggt gaa aaa agt agg agt cgc    144
Asn Asn Pro Tyr Ala Asn Leu Ser Thr Gly Glu Lys Ser Arg Ser Arg
        35                  40                  45

cat aac act ggt agt tct tat gtt tct cca tat ggc ggc ggt aat gga    192
His Asn Thr Gly Ser Ser Tyr Val Ser Pro Tyr Gly Gly Gly Asn Gly
    50                  55                  60

gag gag aat gct tat act ggg aat aac aac aaa tca aat act agt ggt    240
Glu Glu Asn Ala Tyr Thr Gly Asn Asn Asn Lys Ser Asn Thr Ser Gly
65                  70                  75                  80

aat tta tta caa gtt cct gga gca gga gga gga gga gat ttg aat tct    288
Asn Leu Leu Gln Val Pro Gly Ala Gly Gly Gly Gly Asp Leu Asn Ser
                85                  90                  95

aat aag aaa caa agt cga aga atg agt att cat gta tca gct cgt caa    336
Asn Lys Lys Gln Ser Arg Arg Met Ser Ile His Val Ser Ala Arg Gln
            100                 105                 110

cat gga aga tca ttt tca caa act ggt cca att gat atg gca aat tta    384
His Gly Arg Ser Phe Ser Gln Thr Gly Pro Ile Asp Met Ala Asn Leu
        115                 120                 125

ccg gca tta cct aaa ata ggt ggt gtt act act agt ggt gtt ggc ggt    432
Pro Ala Leu Pro Lys Ile Gly Gly Val Thr Thr Ser Gly Val Gly Gly
    130                 135                 140

gct ggt ggt gat gtt atg aca agg act ggg gga ttg acg ata gaa caa    480
Ala Gly Gly Asp Val Met Thr Arg Thr Gly Gly Leu Thr Ile Glu Gln
145                 150                 155                 160

aaa ata ttc aaa gaa tta agt caa gga tca gca gct gaa gtt gat gat    528
Lys Ile Phe Lys Glu Leu Ser Gln Gly Ser Ala Ala Glu Val Asp Asp
                165                 170                 175

tat tac aag aca tta ttg aaa cag aaa aat tta atc act cgt gac att    576
Tyr Tyr Lys Thr Leu Leu Lys Gln Lys Asn Leu Ile Thr Arg Asp Ile
            180                 185                 190

aag gat aat att aat cag aat caa aaa aat att tta caa tta aca aaa    624
Lys Asp Asn Ile Asn Gln Asn Gln Lys Asn Ile Leu Gln Leu Thr Lys
        195                 200                 205

gac ttg aaa gag acc caa gaa gaa ttg att gaa ttg aga gga acc act    672
Asp Leu Lys Glu Thr Gln Glu Glu Leu Ile Glu Leu Arg Gly Thr Thr
    210                 215                 220

aaa gaa tta tat gaa gtt tta ggt tat ttc aaa gaa tca gct caa cgt    720
Lys Glu Leu Tyr Glu Val Leu Gly Tyr Phe Lys Glu Ser Ala Gln Arg
225                 230                 235                 240

aga tta gaa ttg gaa ttt gaa cca gaa aca caa aaa gaa ctt cat ctg    768
Arg Leu Glu Leu Glu Phe Glu Pro Glu Thr Gln Lys Glu Leu His Leu
                245                 250                 255

cct caa aaa agt aat caa ttg ggt att cct agt aat aaa aag aaa gat    816
Pro Gln Lys Ser Asn Gln Leu Gly Ile Pro Ser Asn Lys Lys Lys Asp
            260                 265                 270

cga tca tca att atg gtg ctt aaa aaa atg tgg gat tct caa tta caa    864

Arg Ser Ser Ile Met Val Leu Lys Lys Met Trp Asp Ser Gln Leu Gln
        275                 280                 285

tca tta ttt aaa cat gtt gac ggt gca tca aaa ttt gtc caa cca tta     912
Ser Leu Phe Lys His Val Asp Gly Ala Ser Lys Phe Val Gln Pro Leu
    290                 295                 300

ccc aat aga cac att gtc gcg gaa agt gga cga tgg ttt gaa gtt aat     960
Pro Asn Arg His Ile Val Ala Glu Ser Gly Arg Trp Phe Glu Val Asn
305                 310                 315                 320

gtg ggg aat tgg aaa cca agt tat cca act cat tta ttt ata ttt aat     1008
Val Gly Asn Trp Lys Pro Ser Tyr Pro Thr His Leu Phe Ile Phe Asn
                325                 330                 335

gat tta att tta att act gtt aaa aaa tca tca tct agt agt cag gaa     1056
Asp Leu Ile Leu Ile Thr Val Lys Lys Ser Ser Ser Ser Ser Gln Glu
            340                 345                 350

cct act aca ggg gga agt aat ggt ggt tca aaa tcg aga tta caa gcg     1104
Pro Thr Thr Gly Gly Ser Asn Gly Gly Ser Lys Ser Arg Leu Gln Ala
        355                 360                 365

gtt caa tgt tgg ccc tta act caa gta tca tta caa caa atc aaa tca     1152
Val Gln Cys Trp Pro Leu Thr Gln Val Ser Leu Gln Gln Ile Lys Ser
    370                 375                 380

ccg aaa aaa gat gac gat aag atg tat ttt atc aat ctt aaa tcc aaa     1200
Pro Lys Lys Asp Asp Asp Lys Met Tyr Phe Ile Asn Leu Lys Ser Lys
385                 390                 395                 400

tct tta agt tat gta tac ctg acg gat cgt tat gat cat ttt gtg aaa     1248
Ser Leu Ser Tyr Val Tyr Leu Thr Asp Arg Tyr Asp His Phe Val Lys
                405                 410                 415

gtt acg gaa gca ttt aat aaa ggt aga aat gaa atg att caa agt gaa     1296
Val Thr Glu Ala Phe Asn Lys Gly Arg Asn Glu Met Ile Gln Ser Glu
            420                 425                 430

aga tta tta gat tca aga ctt tca tct cct tca aat aat aat gga gat     1344
Arg Leu Leu Asp Ser Arg Leu Ser Ser Pro Ser Asn Asn Asn Gly Asp
        435                 440                 445

tct aaa gaa gag aaa cga caa tta cgg gaa tca tta aga aac tca ggc     1392
Ser Lys Glu Glu Lys Arg Gln Leu Arg Glu Ser Leu Arg Asn Ser Gly
    450                 455                 460

aat tat aaa gaa gga gtt act gat gat gcc ggt gga gct gca act ggt     1440
Asn Tyr Lys Glu Gly Val Thr Asp Asp Ala Gly Gly Ala Ala Thr Gly
465                 470                 475                 480

ggt ggt agg aaa agt gcc ggt act cct aat aga aat agt act gat tac     1488
Gly Gly Arg Lys Ser Ala Gly Thr Pro Asn Arg Asn Ser Thr Asp Tyr
                485                 490                 495

gtt tta cat gat ata tct gct cga gta cat tca cgt aat cga tca caa     1536
Val Leu His Asp Ile Ser Ala Arg Val His Ser Arg Asn Arg Ser Gln
            500                 505                 510

gat tta ggg aat aat ttc aaa tta gct aat aat ggg aaa tca caa ttt     1584
Asp Leu Gly Asn Asn Phe Lys Leu Ala Asn Asn Gly Lys Ser Gln Phe
        515                 520                 525

ttc aat gaa atc aaa act tta gaa gat cga tta gat gat gtt gac gtt     1632
Phe Asn Glu Ile Lys Thr Leu Glu Asp Arg Leu Asp Asp Val Asp Val

530                 535                 540

gaa ata tcg cat aat caa tat gct gaa gcc gtg gaa tta ata tca ata     1680
Glu Ile Ser His Asn Gln Tyr Ala Glu Ala Val Glu Leu Ile Ser Ile
545                 550                 555                 560

att gaa tct aaa tta cgt aat att gaa aat gca tta act aat caa cgt     1728
Ile Glu Ser Lys Leu Arg Asn Ile Glu Asn Ala Leu Thr Asn Gln Arg
                565                 570                 575

aat gga ggt aaa aat gtc aat att gct gat gaa tta tta ctt tta gat     1776
Asn Gly Gly Lys Asn Val Asn Ile Ala Asp Glu Leu Leu Leu Leu Asp
            580                 585                 590

gta tca aaa ttg aaa att aaa aat cgg aaa gaa aat gta tct aat gga     1824
Val Ser Lys Leu Lys Ile Lys Asn Arg Lys Glu Asn Val Ser Asn Gly
        595                 600                 605

tta ata ttt gat tta caa cat aat ata gct aaa ctt aaa caa gat gat     1872
Leu Ile Phe Asp Leu Gln His Asn Ile Ala Lys Leu Lys Gln Asp Asp
    610                 615                 620

att gat aat att ttg acg tta ttt gat aat tta gag caa tta gat cga     1920
Ile Asp Asn Ile Leu Thr Leu Phe Asp Asn Leu Glu Gln Leu Asp Arg
625                 630                 635                 640

ggg gtt caa gga tat ttg gat tca atg tca gct tat tta tca act aca     1968
Gly Val Gln Gly Tyr Leu Asp Ser Met Ser Ala Tyr Leu Ser Thr Thr
                645                 650                 655

gta tca aaa tta att gtt ggt tta caa gga tca acg aaa atc gat gtt     2016
Val Ser Lys Leu Ile Val Gly Leu Gln Gly Ser Thr Lys Ile Asp Val
            660                 665                 670

gtt aat tat ctt tcc aat tta atg gtt att aat gta tcg att gtg aaa     2064
Val Asn Tyr Leu Ser Asn Leu Met Val Ile Asn Val Ser Ile Val Lys
        675                 680                 685

cgt aca att caa act tat gaa caa ata att gct cca att tta aaa cgt     2112
Arg Thr Ile Gln Thr Tyr Glu Gln Ile Ile Ala Pro Ile Leu Lys Arg
    690                 695                 700

cat ggt gat gtt gat tca agt gga ttg att aat tgg tgt att gat gaa     2160
His Gly Asp Val Asp Ser Ser Gly Leu Ile Asn Trp Cys Ile Asp Glu
705                 710                 715                 720

ttt act aaa ctt tgt aaa caa att aaa aaa cat ttg tat gga aca ttg     2208
Phe Thr Lys Leu Cys Lys Gln Ile Lys Lys His Leu Tyr Gly Thr Leu
                725                 730                 735

ttg ata tct tct ggg att aat atg gaa act gat gaa cca att tat aaa     2256
Leu Ile Ser Ser Gly Ile Asn Met Glu Thr Asp Glu Pro Ile Tyr Lys
            740                 745                 750

gtt aaa gaa aga aaa tta tat gat aat ttc ttg aag att atg caa cca     2304
Val Lys Glu Arg Lys Leu Tyr Asp Asn Phe Leu Lys Ile Met Gln Pro
        755                 760                 765

caa ttg gaa gaa tta aaa ctg gtg gga tta aat gtt gat tat ata ttt     2352
Gln Leu Glu Glu Leu Lys Leu Val Gly Leu Asn Val Asp Tyr Ile Phe
    770                 775                 780

gag tct ata tta aat ctt gaa                                         2373
Glu Ser Ile Leu Asn Leu Glu
785                 790

<210> 6
<211> 791
<212> PRT
<213> Candida albicans

<400> 6
Met Asp Asn Leu Asp Pro Asn Ser Ser Leu Gln Val Glu Lys Leu Arg
1               5                   10                  15

Asn Arg Lys Ser Arg Ala Val Trp Gln Asn Asn Asn Thr Ser Thr His
            20                  25                  30

Asn Asn Pro Tyr Ala Asn Leu Ser Thr Gly Glu Lys Ser Arg Ser Arg
        35                  40                  45

His Asn Thr Gly Ser Ser Tyr Val Ser Pro Tyr Gly Gly Gly Asn Gly
    50                  55                  60

Glu Glu Asn Ala Tyr Thr Gly Asn Asn Asn Lys Ser Asn Thr Ser Gly
65                  70                  75                  80

Asn Leu Leu Gln Val Pro Gly Ala Gly Gly Gly Gly Asp Leu Asn Ser
                85                  90                  95

Asn Lys Lys Gln Ser Arg Arg Met Ser Ile His Val Ser Ala Arg Gln
            100                 105                 110

His Gly Arg Ser Phe Ser Gln Thr Gly Pro Ile Asp Met Ala Asn Leu
        115                 120                 125

Pro Ala Leu Pro Lys Ile Gly Gly Val Thr Thr Ser Gly Val Gly Gly
    130                 135                 140

Ala Gly Gly Asp Val Met Thr Arg Thr Gly Gly Leu Thr Ile Glu Gln
145                 150                 155                 160

Lys Ile Phe Lys Glu Leu Ser Gln Gly Ser Ala Ala Glu Val Asp Asp
                165                 170                 175

Tyr Tyr Lys Thr Leu Leu Lys Gln Lys Asn Leu Ile Thr Arg Asp Ile
            180                 185                 190

Lys Asp Asn Ile Asn Gln Asn Gln Lys Asn Ile Leu Gln Leu Thr Lys
        195                 200                 205

Asp Leu Lys Glu Thr Gln Glu Glu Leu Ile Glu Leu Arg Gly Thr Thr
    210                 215                 220

Lys Glu Leu Tyr Glu Val Leu Gly Tyr Phe Lys Glu Ser Ala Gln Arg
225                 230                 235                 240

Arg Leu Glu Leu Glu Phe Glu Pro Glu Thr Gln Lys Glu Leu His Leu
                245                 250                 255

Pro Gln Lys Ser Asn Gln Leu Gly Ile Pro Ser Asn Lys Lys Lys Asp
            260                 265                 270

Arg Ser Ser Ile Met Val Leu Lys Lys Met Trp Asp Ser Gln Leu Gln
        275                 280                 285

Ser Leu Phe Lys His Val Asp Gly Ala Ser Lys Phe Val Gln Pro Leu
    290                 295                 300

Pro Asn Arg His Ile Val Ala Glu Ser Gly Arg Trp Phe Glu Val Asn

305                 310                 315                 320

Val Gly Asn Trp Lys Pro Ser Tyr Pro Thr His Leu Phe Ile Phe Asn
                325                 330                 335

Asp Leu Ile Leu Ile Thr Val Lys Lys Ser Ser Ser Ser Ser Gln Glu
            340                 345                 350

Pro Thr Thr Gly Gly Ser Asn Gly Gly Ser Lys Ser Arg Leu Gln Ala
        355                 360                 365

Val Gln Cys Trp Pro Leu Thr Gln Val Ser Leu Gln Gln Ile Lys Ser
    370                 375                 380

Pro Lys Lys Asp Asp Asp Lys Met Tyr Phe Ile Asn Leu Lys Ser Lys
385                 390                 395                 400

Ser Leu Ser Tyr Val Tyr Leu Thr Asp Arg Tyr Asp His Phe Val Lys
                405                 410                 415

Val Thr Glu Ala Phe Asn Lys Gly Arg Asn Glu Met Ile Gln Ser Glu
            420                 425                 430

Arg Leu Leu Asp Ser Arg Leu Ser Ser Pro Ser Asn Asn Asn Gly Asp
        435                 440                 445

Ser Lys Glu Glu Lys Arg Gln Leu Arg Glu Ser Leu Arg Asn Ser Gly
    450                 455                 460

Asn Tyr Lys Glu Gly Val Thr Asp Asp Ala Gly Gly Ala Ala Thr Gly
465                 470                 475                 480

Gly Gly Arg Lys Ser Ala Gly Thr Pro Asn Arg Asn Ser Thr Asp Tyr
                485                 490                 495

Val Leu His Asp Ile Ser Ala Arg Val His Ser Arg Asn Arg Ser Gln
            500                 505                 510

Asp Leu Gly Asn Asn Phe Lys Leu Ala Asn Asn Gly Lys Ser Gln Phe
        515                 520                 525

Phe Asn Glu Ile Lys Thr Leu Glu Asp Arg Leu Asp Asp Val Asp Val
    530                 535                 540

Glu Ile Ser His Asn Gln Tyr Ala Glu Ala Val Glu Leu Ile Ser Ile
545                 550                 555                 560

Ile Glu Ser Lys Leu Arg Asn Ile Glu Asn Ala Leu Thr Asn Gln Arg
                565                 570                 575

Asn Gly Gly Lys Asn Val Asn Ile Ala Asp Glu Leu Leu Leu Leu Asp
            580                 585                 590

Val Ser Lys Leu Lys Ile Lys Asn Arg Lys Glu Asn Val Ser Asn Gly
        595                 600                 605

Leu Ile Phe Asp Leu Gln His Asn Ile Ala Lys Leu Lys Gln Asp Asp
    610                 615                 620

Ile Asp Asn Ile Leu Thr Leu Phe Asp Asn Leu Glu Gln Leu Asp Arg
625                 630                 635                 640

Gly Val Gln Gly Tyr Leu Asp Ser Met Ser Ala Tyr Leu Ser Thr Thr
                645                 650                 655

Val Ser Lys Leu Ile Val Gly Leu Gln Gly Ser Thr Lys Ile Asp Val
            660                 665                 670

Val Asn Tyr Leu Ser Asn Leu Met Val Ile Asn Val Ser Ile Val Lys
        675                 680                 685

Arg Thr Ile Gln Thr Tyr Glu Gln Ile Ile Ala Pro Ile Leu Lys Arg
    690                 695                 700

His Gly Asp Val Asp Ser Ser Gly Leu Ile Asn Trp Cys Ile Asp Glu
705                 710                 715                 720

Phe Thr Lys Leu Cys Lys Gln Ile Lys Lys His Leu Tyr Gly Thr Leu
                725                 730                 735

Leu Ile Ser Ser Gly Ile Asn Met Glu Thr Asp Glu Pro Ile Tyr Lys
            740                 745                 750

Val Lys Glu Arg Lys Leu Tyr Asp Asn Phe Leu Lys Ile Met Gln Pro
        755                 760                 765

Gln Leu Glu Glu Leu Lys Leu Val Gly Leu Asn Val Asp Tyr Ile Phe
    770                 775                 780

Glu Ser Ile Leu Asn Leu Glu
785                 790


<210> 7
<211> 1248
<212> DNA
<213> Candida albicans

<220>
<221> CDS
<222> (1)..(1245)

<220>
<221> gene
<222> (1)..(1245)
<223> gene CaIR012

<400> 7
atg tca cac caa caa gaa gac gtc gta gac gat act caa gaa gaa tat      48
Met Ser His Gln Gln Glu Asp Val Val Asp Asp Thr Gln Glu Glu Tyr
1               5                   10                  15

atc aat gtt aat gaa gtg gct gag gaa gtt gca gat gat gat caa gcg      96
Ile Asn Val Asn Glu Val Ala Glu Glu Val Ala Asp Asp Asp Gln Ala
            20                  25                  30

cca ccc gat gaa gaa gat gag gag atg gaa tta gat gat gag cat gag      144
Pro Pro Asp Glu Glu Asp Glu Glu Met Glu Leu Asp Asp Glu His Glu
        35                  40                  45

act tta gaa att gac atg tcc aac aat tca tgg act tat ttt gat aaa      192
Thr Leu Glu Ile Asp Met Ser Asn Asn Ser Trp Thr Tyr Phe Asp Lys
    50                  55                  60

cat acc gat agt ata ttt act att ttt tca cat cct aaa ttg cca atg      240
His Thr Asp Ser Ile Phe Thr Ile Phe Ser His Pro Lys Leu Pro Met
65                  70                  75                  80

gta ttg act ggg ggt ggt gac aac acg gca tac tta tgg acc aca cac      288
Val Leu Thr Gly Gly Gly Asp Asn Thr Ala Tyr Leu Trp Thr Thr His

                85                  90                  95

acc caa cca cca aga ttt gtt ggc gaa atc act gga cat aaa gag tct 336
Thr Gln Pro Pro Arg Phe Val Gly Glu Ile Thr Gly His Lys Glu Ser
            100                 105                 110

gtt ata tct gga ggg ttt act gca gac ggc aag ttt gtt gtt act gca 384
Val Ile Ser Gly Gly Phe Thr Ala Asp Gly Lys Phe Val Val Thr Ala
        115                 120                 125

gac atg aat gga tta att caa gtt ttc aaa gcc aca aaa gga ggt gaa 432
Asp Met Asn Gly Leu Ile Gln Val Phe Lys Ala Thr Lys Gly Gly Glu
    130                 135                 140

cag tgg gtg aaa ttt ggt gaa ttg gac gaa gtt gaa gaa gtg ttg ttt 480
Gln Trp Val Lys Phe Gly Glu Leu Asp Glu Val Glu Glu Val Leu Phe
145                 150                 155                 160

gtt act gtg cat cca aca tta cca ttc ttt gcc ttt ggt gct acc gat 528
Val Thr Val His Pro Thr Leu Pro Phe Phe Ala Phe Gly Ala Thr Asp
                165                 170                 175

gga tct ata tgg gtc tac caa ata gac gaa tcc agt aaa ctg cta gtg 576
Gly Ser Ile Trp Val Tyr Gln Ile Asp Glu Ser Ser Lys Leu Leu Val
            180                 185                 190

caa att atg tct ggg ttc tca cac aca tta gaa tgt aat ggt gct gta 624
Gln Ile Met Ser Gly Phe Ser His Thr Leu Glu Cys Asn Gly Ala Val
        195                 200                 205

ttt ata caa gga aaa gat gaa aat gat ttg aca ttg gtc tct ata agt 672
Phe Ile Gln Gly Lys Asp Glu Asn Asp Leu Thr Leu Val Ser Ile Ser
    210                 215                 220

gaa gat ggt act gtg gtg aac tgg aac tgt ttt aca gga caa gtg aat 720
Glu Asp Gly Thr Val Val Asn Trp Asn Cys Phe Thr Gly Gln Val Asn
225                 230                 235                 240

tat aaa ttg caa cct cat gat gac ttt aaa gga gtt gaa agt ccg tgg 768
Tyr Lys Leu Gln Pro His Asp Asp Phe Lys Gly Val Glu Ser Pro Trp
                245                 250                 255

gtc acg gtc aaa gta cat ggt aat ctt gtg gcc att ggt ggc aga gat 816
Val Thr Val Lys Val His Gly Asn Leu Val Ala Ile Gly Gly Arg Asp
            260                 265                 270

ggc cag cta tca att gtg aac aat gac act ggt aaa atc gtt cat act 864
Gly Gln Leu Ser Ile Val Asn Asn Asp Thr Gly Lys Ile Val His Thr
        275                 280                 285

ctt aaa aca ttg gat aat gtc gac gac att gca gaa ctc tca att gag 912
Leu Lys Thr Leu Asp Asn Val Asp Asp Ile Ala Glu Leu Ser Ile Glu
    290                 295                 300

gca ttg agt tgg tgt gaa agc aaa aat att aac ctc ttg gca gtg ggt 960
Ala Leu Ser Trp Cys Glu Ser Lys Asn Ile Asn Leu Leu Ala Val Gly
305                 310                 315                 320

ttg gtt tct ggt gac gtt tta tta ttt gat act cag caa tgg aga ttg 1008
Leu Val Ser Gly Asp Val Leu Leu Phe Asp Thr Gln Gln Trp Arg Leu
                325                 330                 335

aga aag aac ttg aaa gtt gac gat gcc atc acc aaa tta caa ttt gtt 1056
Arg Lys Asn Leu Lys Val Asp Asp Ala Ile Thr Lys Leu Gln Phe Val
            340                 345                 350

ggc gaa acc ccc att ttg gtg gga agt agt atg gat ggt aaa att tac 1104
Gly Glu Thr Pro Ile Leu Val Gly Ser Ser Met Asp Gly Lys Ile Tyr
        355                 360                 365

aaa tgg gac gct aga act ggt gaa gag ttg ttt gct ggt gtg gga cac 1152
Lys Trp Asp Ala Arg Thr Gly Glu Glu Leu Phe Ala Gly Val Gly His
    370                 375                 380

aac atg gga gta ttg gac ttt gct att tta gat gga ggt aaa aag ttg 1200
Asn Met Gly Val Leu Asp Phe Ala Ile Leu Asp Gly Gly Lys Lys Leu
385                 390                 395                 400

gtt act gct ggt gat gaa ggt gtt tca ttg gtc ttt gta cat gaa tag 1248
Val Thr Ala Gly Asp Glu Gly Val Ser Leu Val Phe Val His Glu
                405                 410                 415

<210> 8
<211> 415
<212> PRT
<213> Candida albicans

<400> 8
Met Ser His Gln Gln Glu Asp Val Val Asp Asp Thr Gln Glu Glu Tyr
1               5                   10                  15

Ile Asn Val Asn Glu Val Ala Glu Glu Val Ala Asp Asp Asp Gln Ala
            20                  25                  30

Pro Pro Asp Glu Glu Asp Glu Glu Met Glu Leu Asp Asp Glu His Glu
        35                  40                  45

Thr Leu Glu Ile Asp Met Ser Asn Asn Ser Trp Thr Tyr Phe Asp Lys
    50                  55                  60

His Thr Asp Ser Ile Phe Thr Ile Phe Ser His Pro Lys Leu Pro Met
65                  70                  75                  80

Val Leu Thr Gly Gly Gly Asp Asn Thr Ala Tyr Leu Trp Thr Thr His
                85                  90                  95

Thr Gln Pro Pro Arg Phe Val Gly Glu Ile Thr Gly His Lys Glu Ser
            100                 105                 110

Val Ile Ser Gly Gly Phe Thr Ala Asp Gly Lys Phe Val Val Thr Ala
        115                 120                 125

Asp Met Asn Gly Leu Ile Gln Val Phe Lys Ala Thr Lys Gly Gly Glu
    130                 135                 140

Gln Trp Val Lys Phe Gly Glu Leu Asp Glu Val Glu Glu Val Leu Phe
145                 150                 155                 160

Val Thr Val His Pro Thr Leu Pro Phe Phe Ala Phe Gly Ala Thr Asp
                165                 170                 175

Gly Ser Ile Trp Val Tyr Gln Ile Asp Glu Ser Ser Lys Leu Leu Val
            180                 185                 190

Gln Ile Met Ser Gly Phe Ser His Thr Leu Glu Cys Asn Gly Ala Val
        195                 200                 205

Phe Ile Gln Gly Lys Asp Glu Asn Asp Leu Thr Leu Val Ser Ile Ser
    210                 215                 220

Glu Asp Gly Thr Val Val Asn Trp Asn Cys Phe Thr Gly Gln Val Asn
225                 230                 235                 240

Tyr Lys Leu Gln Pro His Asp Asp Phe Lys Gly Val Glu Ser Pro Trp
                245                 250                 255

Val Thr Val Lys Val His Gly Asn Leu Val Ala Ile Gly Gly Arg Asp
            260                 265                 270

Gly Gln Leu Ser Ile Val Asn Asn Asp Thr Gly Lys Ile Val His Thr
        275                 280                 285

Leu Lys Thr Leu Asp Asn Val Asp Asp Ile Ala Glu Leu Ser Ile Glu
    290                 295                 300

Ala Leu Ser Trp Cys Glu Ser Lys Asn Ile Asn Leu Leu Ala Val Gly
305                 310                 315                 320

Leu Val Ser Gly Asp Val Leu Leu Phe Asp Thr Gln Gln Trp Arg Leu
                325                 330                 335

Arg Lys Asn Leu Lys Val Asp Asp Ala Ile Thr Lys Leu Gln Phe Val
            340                 345                 350

Gly Glu Thr Pro Ile Leu Val Gly Ser Ser Met Asp Gly Lys Ile Tyr
        355                 360                 365

Lys Trp Asp Ala Arg Thr Gly Glu Glu Leu Phe Ala Gly Val Gly His
    370                 375                 380

Asn Met Gly Val Leu Asp Phe Ala Ile Leu Asp Gly Gly Lys Lys Leu
385                 390                 395                 400

Val Thr Ala Gly Asp Glu Gly Val Ser Leu Val Phe Val His Glu
                405                 410                 415

<210> 9
<211> 647
<212> DNA
<213> Artificial Sequence

<220>
<223> Description of Artificial Sequence: probe

<400> 9
agtattcaat tgggtattcc cagtaataaa aagaaagatc gatcatcaat tatggtgctt 60

aaaaaaatgt gggattctca attacaatca ttatttaaac atgttgacgg tgcatcaaaa 120

tttgtgcaac cattacccaa tagacacatt gtcgcggaaa gtggacgatg gtttgaagtt 180

aatgtgggga attggaaacc aagttatcca actcatttat ttatatttaa tgatttaatt 240

ttaattgccg ttaaaaaatc atcatctagt agtcaggaac ctactacagg gggaagtaat 300

ggtggttcaa aatcgagatt acaagcggtt caatgttggc ccttaactca agtatcatta 360

caacaaatca aatcaccgaa aaaagatgac gataagatgt attttatcaa tcttaaatcc 420

aaatctttaa gttatgtata cctgacggat cgttatgatc attttgtgaa agttacggaa 480

gcatttaata aaggtagaaa tgaaatgatt caaagtgaaa gattattaga ttcaagactt 540

tcatctcctt caaataataa tggagattct aaagaagaga aacgacaatt acgggaatca 600

ttaagaaact caggcaatta taaagaagga gttactgatg atgccgg 647